(19) Europäisches Patentamt
European Patent Office
Office européen des brevets

(11) EP 1 938 891 A1

(12) **DEMANDE DE BREVET EUROPEEN**

(43) Date de publication:
**02.07.2008 Bulletin 2008/27**

(51) Int Cl.:
***B01J 13/14*** *(2006.01)*

(21) Numéro de dépôt: **07123545.1**

(22) Date de dépôt: **19.12.2007**

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité: **20.12.2006 FR 0655678**

(71) Demandeur: **L'ORÉAL**
**75008 Paris (FR)**

(72) Inventeur: **Prigent, Fanny**
**75019 PARIS (FR)**

(74) Mandataire: **Le Coupanec, Pascale A.M.P.**
**Nony & Associés,**
**3 rue de Penthièvre**
**75008 Paris (FR)**

(54) **Particules coeur/écorce à base de composés siliconés**

(57) La présente invention concerne des particules de type coeur/écorce appropriées à l'encapsulation d'actifs lipophiles cosmétiques et/ou thérapeutiques. Elle vise en outre un procédé de préparation desdites particules ainsi que les compositions cosmétiques ou thérapeutiques les comprenant.

EP 1 938 891 A1

## Description

La présente invention concerne des particules de type coeur/écorce appropriées à l'encapsulation d'actifs lipophiles cosmétiques et/ou thérapeutiques. Elle vise en outre un procédé de préparation desdites particules ainsi que les compositions cosmétiques ou thérapeutiques les comprenant.

Depuis longtemps, il est usuel de mettre en oeuvre, dans des compositions dédiées au soin cosmétique ou thérapeutique, des actifs sous une forme encapsulée. Ce type de formulation est généralement privilégié lorsque l'on cherche à prévenir toute dégradation potentielle de l'actif considéré, susceptible d'intervenir par exemple suite à un contact de cet actif sous une forme non encapsulée avec un autre composant de la composition le contenant ou, plus généralement, consécutivement à une exposition prolongée de la composition le contenant à l'atmosphère ambiante. L'encapsulation de ces actifs permet ainsi de renforcer efficacement leur activité. Des exemples de particules creuses de type microcapsules et nanocapsules sont notamment décrits dans les documents EP-A-557489, EP-A-447318, WO-A-93/25195 et WO-A-93/05753.

Ces particules se présentent généralement selon une architecture coeur/écorce. Le coeur est le plus souvent de nature huileuse et contient l'actif lipophile, à savoir une huile ou un composé lipophile distinct d'une huile. Quant à l'écorce, elle est généralement de nature organique et plus particulièrement polymérique.

Toutefois, il est également connu que certaines de ces particules peuvent être sujettes à des phénomènes de relargage non contrôlé par exemple intempestif ou encore qualifiable de parasitaire dans la mesure où il s'agit d'un relargage à faible échelle mais perpétuel. En fait, ce type de relargage est généralement provoqué par une fluidification partielle du polymère formant l'écorce des particules. Cette fluidification peut être notamment la conséquence d'une exposition des particules à une hausse de température.

Pour des raisons évidentes, ce défaut de stabilité, manifesté par certains polymères, est particulièrement préjudicable sur le plan de l'efficacité.

La présente invention a précisément pour objet de proposer de nouvelles particules propices à l'encapsulation d'actifs et qui soient en particulier dénuées de l'inconvénient précité.

En conséquence, la présente invention concerne selon un de ses aspects, des particules de type coeur/écorce à coeur lipophile caractérisées en ce que leur écorce est formée au moins en partie d'un film polymérique siliconé qui est le produit de la réaction *in situ* entre au moins un composé X et au moins un composé Y avec au moins l'un des composés X et Y étant un polyorganosiloxane et ladite réaction étant de type hydrosilylation réalisée en présence d'un catalyseur, ou condensation, ou réticulation réalisée en présence d'un peroxyde.

Au sens de la présente invention, l'expression « réaction *in situ* » signifie que la réaction entre les composés X et Y intervient au moment de la formation des particules.

Avantageusement, le coeur des particules comprend au moins une huile et/ou un agent actif cosmétique ou thérapeutique lipophile.

Selon un mode de réalisation, il peut s'agir de particules dont l'écorce est en outre revêtue au moins en partie d'une phase lamellaire.

Selon un autre de ses aspects, la présente invention concerne une dispersion aqueuse desdites particules.

Selon encore un autre de ses aspects, la présente invention concerne des compositions cosmétiques et/ou thérapeutiques, notamment destinées au soin et/ou maquillage de matière(s) kératinique(s), contenant dans un milieu physiologiquement acceptable au moins une particule conforme à l'invention.

Selon encore un autre de ses aspects, la présente invention se rapporte à l'utilisation cosmétique desdites particules à titre de véhicule d'actif(s) lipophile(s) dans une composition notamment cosmétique et plus particulièrement pour le soin de la peau, la photoprotection et également le maquillage de matière(s) kératinique(s).

Selon encore un autre de ses aspects, la présente invention se rapporte à l'utilisation desdites particules à titre de véhicule d'actif(s) lipophile(s) pour la préparation de compositions, notamment thérapeutiques.

Elle vise en outre une méthode de traitement cosmétique de matière(s) kératinique(s) comprenant au moins l'application sur une matière kératinique de particules ou d'une composition conforme(s) à l'invention.

Selon encore un autre de ses aspects, la présente invention concerne un procédé de préparation de particules de type coeur/écorce dont le coeur est lipophile et l'écorce est formée au moins en partie d'un film polymérique siliconé, ledit procédé comprenant au moins les étapes consistant à

1) solubiliser dans au moins un solvant organique volatil, au moins un composé lipophile, un composé X et un composé Y, avec au moins l'un des composés X et Y étant un polyorganosiloxane et les composés X et Y étant dans des conditions non propices à leur interaction et,
2) mettre en présence la phase organique de l'étape 1 avec une phase aqueuse contenant le cas échéant au moins un tensio-actif, dans des conditions propices à la formation de gouttelettes lipophiles et à la réaction des composés X et Y selon une réaction de type hydrosilylation en présence d'un catalyseur, condensation ou réticulation en présence d'un peroxyde pour former un film polymérique siliconé en surface desdites gouttelettes.

Les particules ainsi formées peuvent être isolées par évaporation du ou des solvants organiques.

Selon un mode de réalisation, l'étape 2 est réalisée en présence d'un catalyseur s'il est nécessaire à l'interaction des composés X et Y.

Selon un autre mode de réalisation particulier, la phase organique de l'étape 1 peut comprendre en outre un composé apte à former un enrobage de type phase lamellaire en surface des particules attendues et/ou un agent actif lipophile annexe c'est-à-dire distinct du composé lipophile.

Selon une première variante de réalisation, le solvant organique est faiblement miscible à l'eau, comme par exemple le dichlorométhane et l'étape 2 correspond à une émulsification de la phase organique de l'étape 1 au sein de la phase aqueuse à l'état de gouttelettes en surface desquelles les composés X et Y interagissent pour former le film polymérique constituant l'écorce des particules correspondantes.

Cette technologie de préparation de particules est notamment décrite dans le brevet FR 2 864 900.

Selon une seconde variante de réalisation, le solvant organique est miscible à l'eau et l'étape 2 correspond à un basculement de solvant au cours duquel la diffusion de la phase aqueuse au sein de la phase organique provoque la formation de gouttelettes en surface desquelles les composés X et Y interagissent pour produire le film polymérique formant l'écorce des particules correspondantes.

Cette technologie de préparation de particules est notamment décrite dans le document EP 0 274 961.

De manière inattendue et comme le démontrent les exemples ci-après, les inventeurs ont ainsi constaté qu'un film polymérique produit par réaction *in situ* d'un composé X avec au moins un composé Y tels que définis ci-après, c'est-à-dire dont la formation intervient en même temps que celle des particules de type coeur/écorce dont il constitue l'écorce, conduit à des particules présentant une stabilité accrue dans le temps.

Les composés X et Y, plus précisément décrits ci-après, sont des composés connus pour interagir généralement à température ambiante et pression atmosphérique et former ainsi des films polymériques siliconés. Ces composés sont en partie décrits dans les documents WO 01/96450 et GB 2 407 496. Toutefois, à la connaissance des inventeurs ces composés n'ont jusqu'à ce jour jamais été mis en oeuvre pour obtenir des particules conformes à l'invention.

## DEFINITION DES PARTICULES

Les particules selon l'invention peuvent posséder un diamètre moyen des particules (ou « taille ») variant de 50nm à 100μm

Ce diamètre peut notamment être contrôlé selon une technique de mesure reposant sur la diffusion dynamique de la lumière avec par exemple un appareil BI90+® de chez Broohaven Instrument ou Malvern Mastersizer 2000 (adapté pour les Ø sup. à 2μm).

Selon une première variante de réalisation, les particules selon l'invention peuvent être des microcapsules et donc posséder un diamètre particulaire moyen allant de 1 μm à 100 μm

Selon une seconde variante, il s'agit de nanocapsules et elles possèdent alors un diamètre particulaire moyen variant de 50nm à 1μm.

Comme précisé précédemment, les particules selon l'invention présentent une architecture de type coeur/écorce et possèdent un coeur de nature lipophile.

## ECORCE DES PARTICULES SELON L'INVENTION

L'écorce des particules selon l'invention est formée au moins en partie, et avantageusement est constituée, d'un film polymérique siliconé *formé in situ* par la réaction d'au moins un composé X et d'au moins un composé Y, le cas échéant en présence d'un catalyseur ou d'un peroxyde l'un au moins de ces deux composés étant un polyorganosiloxane.

### Composés X et Y

Par composé siliconé, on entend un composé polyorganosiloxane, c'est-à-dire comprenant au moins deux unités organosiloxane par exemple au moins 5 unités organosiloxane, notamment au moins 10 unités organosiloxane. Selon un mode de réalisation particulier, l'un au moins des composés X et Y, ou les composés X et les composés Y sont des polyorganosiloxanes. Les composés X et Y peuvent être aminés ou non aminés.

Selon un autre mode de réalisation, au moins un des composés X et Y est un polymère dont la chaîne principale est formée majoritairement d'unités organosiloxanes.

Les inventeurs ont en effet constaté que les composés comprenant une seule unité organosiloxane forment par polymérisation un réseau très rigide, conduisant à des particules présentant des écorces très dures.

Au contraire, en utilisant au moins un composé X ou Y de type polyorganosiloxane, l'écorce des particules résultantes est plus souple, et les compositions comprenant ces particules présentent de meilleures propriétés cosmétiques, notamment une meilleure douceur lors de l'application sur la peau.

Parmi les composés siliconés cités ci-après, certains peuvent présenter à la fois des propriétés filmogènes et adhésives, selon par exemple leur proportion en silicone ou selon qu'on les utilise en mélange avec un additif particulier. Il est par conséquent possible de moduler les propriétés filmogènes ou les propriétés adhésives de tels composés selon l'utilisation envisagée, c'est en particulier le cas pour les silicones élastomères réactives dites « room temperature vulcanization ».

Les composés X et Y peuvent réagir ensemble à une température variant entre la température ambiante et 180°C. Avantageusement les composés X et Y sont susceptibles de réagir ensemble à température ambiante (20 $\pm$ 5°C) et pression atmosphérique, par une réaction d'hydrosilylation en présence d'un catalyseur, ou une réaction de condensation le cas échéant en présence d'un catalyseur, ou une réaction de réticulation en présence d'un peroxyde.

Groupes polaires

Selon un mode de réalisation particulier, l'un au moins des composé X et Y, par exemple le composé X, est porteur d'au moins un groupe polaire susceptible de former au moins une liaison hydrogène avec les matières kératiniques.

Par groupe polaire, on entend un groupe comportant des atomes de carbone et d'hydrogène dans sa structure chimique et au moins un hétéroatome (tel que O, N, S et P), tel que ledit groupe est apte à établir au moins une liaison hydrogène avec les matières kératiniques.

Des composés porteurs d'au moins un groupement apte à établir une liaison hydrogène sont particulièrement avantageux, car ils apportent aux compositions les contenant une meilleure adhérence sur les matières kératiniques.

Le ou les groupe(s) polaire(s) porté(s) par au moins l'un des composés X et Y est/sont apte(s) à établir une liaison hydrogène, et comporte(nt) soit un atome d'hydrogène lié à un atome électronégatif, soit un atome électronégatif comme par exemple l'atome d'oxygène, d'azote ou de soufre. Lorsque le groupe comporte un atome d'hydrogène lié à un atome électronégatif, l'atome d'hydrogène peut interagir avec un autre atome électronégatif porté, par exemple par une autre molécule, telle que la kératine, pour former une liaison hydrogène. Lorsque le groupement comporte un atome électronégatif, l'atome électronégatif peut interagir avec un atome d'hydrogène lié à un atome électronégatif porté, par exemple par une autre molécule, telle que la kératine, pour former une liaison hydrogène.

Avantageusement, ces groupes polaires peuvent être choisis parmi les groupes suivants :

- acides carboxyliques -COOH,
- alcools, tels que : $-CH_2OH$ ou -CH(R)OH, R étant un radial alkyle comprenant de 1 à 6 atomes de carbone,
- amino de formule $-NR_1R_2$, dans laquelle les $R_1$ et $R_2$ identiques ou différents représentent un radial alkyle comprenant de 1 à 6 atomes de carbone ou l'un des $R_1$ ou $R_2$ désigne un atome d'hydrogène, et l'autre des $R_1$ et $R_2$ représente un radical alkyle comprenant de 1 à 6 atomes de carbone,
- pyridino,
- amido de formule -NH-COR' ou -CO-NH-R' dans laquelle R' représente un atome d'hydrogène ou un radial alkyle comprenant de 1 à 6 atomes de carbone,
- pyrrolidino choisi de préférence parmi les groupes de formule :

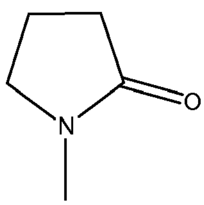 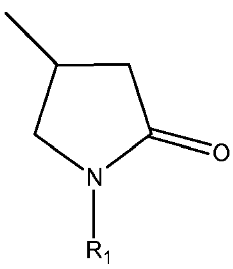 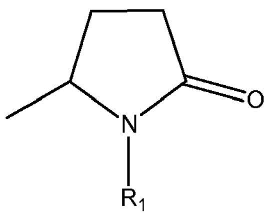 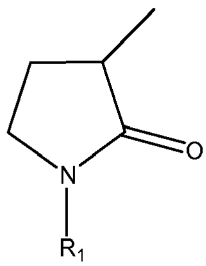

$R_1$ étant un radial alkyle comprenant de 1 à 6 atomes de carbone,

- carbamoyle de formule -O-CO-NH-R' ou -NH-CO-OR', R' étant tel que défini ci-dessus,
- thiocarbamoyle tel que -O-CS-NH- R' ou -NH-CS-OR', R' étant tel que défini ci-dessus,
- uréyle tel que $-NR'-CO-N(R')_2$, les groupes R' identiques ou différents étant tels que définis ci-dessus,
- sulfonamido tel que $-NR'-S(=O)_2-R'$, R' répondant à la définition ci-dessus.

De préférence, ces groupes polaires sont présents à une teneur inférieure ou égale à 10 % en poids par rapport au poids de chaque composé X ou Y, de préférence inférieure ou égale à 5 % en poids, par exemple en une teneur allant de 1 à 3 % en poids.
Le ou les groupes polaires peu(ven)t être situé(s) dans la chaîne principale du composé X et/ou Y ou peuvent être pendants à la chaîne principale ou situés aux extrémités de la chaîne principale du composé X et/ou Y.

**1- Composés X et Y susceptibles de réagir par hydrosilylation**

Selon un mode de réalisation, l'invention concerne des particules de type coeur/écorce à coeur lipophile caractérisées en ce que leur écorce est formée au moins en partie d'un film polymérique siliconé qui est le produit de réaction *in situ* entre au moins un composé X et au moins un composé Y, l'un au moins des composés X et Y étant un polyorganosiloxane et ladite réaction étant de type hydrosilylation réalisée en présence d'un catalyseur.
Selon ce mode de réalisation, les composés X et Y sont susceptibles de réagir par hydrosilylation en présence d'un catalyseur, cette réaction pouvant être de manière simplifiée schématisée comme suit :

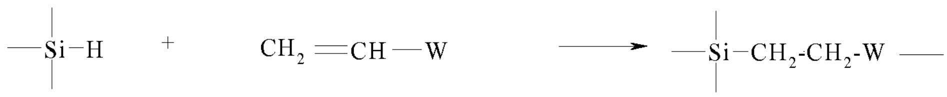

avec W représentant une chaîne carbonée et/ou siliconée contenant un ou plusieurs groupements aliphatiques insaturés.
Dans ce cas, le composé X peut être choisi parmi les composés siliconés comprenant au moins deux groupements aliphatiques insaturés. A titre d'exemple, le composé X peut être un polyorganosiloxane comprenant une chaîne principale siliconée dont les groupements aliphatiques insaturés sont pendants à la chaîne principale (groupe latéral) ou situés aux extrémités de la chaîne principale du composé (groupe terminal). On appellera, dans la suite de la description, ces composés particuliers des polyorganosiloxanes à groupements aliphatiques insaturés.
Selon un mode de réalisation, le composé X et/ou le composé Y est porteur d'au moins un groupe polaire, tel que décrit ci-dessus, susceptible de former au moins une liaison hydrogène avec les matières kératiniques. Ce groupe polaire est avantageusement porté par le composé X qui comprend au moins deux groupements aliphatiques insaturés.
Selon un mode de réalisation, le composé X est choisi parmi les polyorganosiloxanes comprenant au moins deux groupements aliphatique insaturés, par exemple deux ou trois groupements vinyliques ou allyliques, liés chacun à un atome de silicium.
Selon un mode de réalisation avantageux, le composé X est choisi parmi les polyorganosiloxanes comprenant des unités siloxanes de formule :

$$R_m R'SiO_{\frac{(3-m)}{2}} \qquad (I)$$

dans laquelle :

- R représente un groupe hydrocarboné monovalent, linéaire ou cyclique, comprenant de 1 à 30 atomes de carbone, de préférence de 1 à 20, et mieux de 1 à 10 atomes de carbone, comme par exemple un radical alkyle à chaîne courte, comprenant par exemple de 1 à 10 atomes de carbone, en particulier un radical méthyle ou encore un groupement phényle, de préférence un radical méthyle,
- m est égal à 1 ou 2 et
- R' représente :

  o un groupement hydrocarboné aliphatique insaturé comprenant de 2 à 10, de préférence de 3 à 5 atomes de carbone comme par exemple un groupe vinyle ou un groupe -R"-CH=CHR"' dans lequel R" est une chaîne hydrocarbonée aliphatique divalente, comprenant de 1 à 8 atomes de carbone, liée à l'atome de silicium et R"' est un atome d'hydrogène ou un radical alkyle comprenant de 1 à 4 atomes de carbone, de préférence un atome d'hydrogène ; on peut citer comme groupement R' les groupements vinyle, allyle et leurs mélanges ; ou
  o un groupement hydrocarboné cyclique insaturé comprenant de 5 à 8 atomes de carbone comme par exemple un groupe cyclohexènyle.

De préférence R' est un groupement hydrocarboné aliphatique insaturé, de préférence un groupe vinyle.

Selon un mode de réalisation, R représente un radical alkyle comprenant de 1 à 10 atomes de carbone ou encore un groupement phényle, et de préférence un radical méthyle, et R' est un groupe vinyle.

Selon un mode de réalisation particulier, le polyorganosiloxane comprend également des unités de formule :

$$R_nSiO_{\frac{(4-n)}{2}} \quad \text{(II)}$$

dans laquelle R est un groupe tel que défini plus haut, et n est égal à 1, 2 ou 3.

Selon une variante, le composé X peut être une résine de silicone comprenant au moins deux insaturations éthyléniques, ladite résine étant apte à réagir avec le composé Y par hydrosilylation en présence d'un catalyseur. On peut citer par exemple les résines de type MQ ou MT portant elle-même des extrémités réactives insaturées $-CH=CH_2$.

Ces résines sont des polymères d'organosiloxanes réticulés.

La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.

La lettre M représente l'unité monofonctionelle de formule $(CH_3)_3SiO_{1/2}$, l'atome de silicium étant relié à un seul atome d'oxygène dans le polymère comprenant cette unité.

La lettre D signifie une unité difonctionnelle $(CH_3)_2SiO_{2/2}$ dans laquelle l'atome de silicium est relié à deux atomes d'oxygène

La lettre T représente une unité trifonctionnelle de formule $(CH_3)SiO_{3/2}$.

Dans les motifs M, D, T définis précédemment, au moins un des groupes méthyles peut être substitués par un groupe R différent du groupe méthyle tel qu'un radical hydrocarboné (notamment alkyle) ayant de 2 à 10 atomes de carbone ou un groupe phényl

ou bien encore un groupe hydroxyle.

Enfin, la lettre Q signifie une unité tetrafonctionnelle $SiO_{4/2}$ dans laquelle l'atome de silicium est lié à quatre atomes d'hydrogène eux mêmes liés au reste du polymère. Comme exemples de telles résines, on peut citer les résines de silicone MT telles que les poly(phényl-vinylsilsesquioxane) comme celle commercialisées sous la référence SST-3PV1 par la société Gelest.

De préférence, les composés X comprennent de 0,01 à 1 % en poids de groupes aliphatiques insaturés.

Avantageusement, le composé X est choisi parmi les polyorganopolysiloxanes, notamment ceux comprenant les unités siloxanes (I) et éventuellement (II) décrites précédemment.

Le composé Y comprend de préférence au moins deux groupes Si-H (groupes hydrogénosilanes) libres.

Le composé Y peut être avantageusement choisi parmi les polyorganosiloxanes comprenant au moins une unité alkylhydrogènosiloxane de formule suivante :

$$R_pHSiO_{\frac{(3-p)}{2}} \quad \text{(III)}$$

dans laquelle :

R représente un groupe hydrocarboné monovalent, linéaire ou cyclique, comprenant de 1 à 30 atomes de carbone, comme par exemple un radical alkyle ayant de 1 à 30 atomes de carbone, de préférence de 1 à 20 et mieux de 1 à 10 atomes de carbone, en particulier un radical méthyle, ou encore un groupement phényle et p est égal à 1 ou 2. De préférence R est un groupement hydrocarboné, de préférence le méthyle.

Ces composés Y polyorganosiloxanes à unités alkylhydrogènosiloxanes peuvent comprendre en outre des unités de formule :

$$R_nSiO_{\left(\frac{4-n}{2}\right)} \qquad \text{(II)}$$

telles que définies plus haut.

Le composé Y peut être une résine de silicone comprenant au moins un motif choisi parmi les motifs M, D, T, Q tels que définis ci-dessus et comprenant au moins un groupe Si-H tel que les poly(méthyl-hydridosilsesquioxane) commercialisées sous la référence SST-3MH1.1 par la société Gelest.

De préférence, ces composés Y polyorganosiloxanes comprennent de 0,5 à 2,5% en poids de groupes Si-H.

Avantageusement, les radicaux R représentent un groupement méthyle dans les formules (I), (II), (III) ci-dessus.

De préférence, ces polyorganosiloxanes Y comprennent des groupes terminaux de formule $(CH_3)_3SiO_{1/2}$.

Avantageusement, les polyorganosiloxanes Y comprennent au moins deux unités alkylhydrogènosiloxane de formule $-(H_3C)(H)SiO-$ et comprennent éventuellement des unités $-(H_3C)_2SiO-$.

De tels composés polyorganosiloxanes Y à groupements hydrogénosilane sont décrits par exemple dans le document EP 0465744.

Selon une variante, le composé X est choisi parmi les oligomères ou polymères organiques (par organique, on entend des composés dont la chaîne principale est non siliconée, de préférence des composés ne comprenant pas d'atomes de silicium) ou parmi les polymères ou oligomères hybrides organique/silicone, lesdits oligomères ou polymères portant au moins 2 groupements aliphatiques insaturés réactifs, le composé Y étant choisi parmi les polyorganosiloxanes Y à groupements hydrogénosilane cités ci-dessus.

Selon un mode de réalisation, les composés X organiques ou hybrides organique/silicone portant au moins 2 groupements aliphatiques insaturés réactifs, portent au moins un groupe polaire tel que décrit plus haut.

Le composé X, de nature organique, peut alors être choisi parmi les polymères ou oligomères vinyliques, (méth)acryliques, les polyesters, les polyuréthanes et/ou les polyurées, les polyéthers, les perfluoropolyéthers, les polyoléfines telles que le polybutène, le polyisobutylène, les dendrimères ou les polymères hyper-ramifiés organiques, ou leurs mélanges.

En particulier, le polymère organique ou la partie organique du polymère hybride peut être choisi parmi les polymères suivants :

a) les polyesters à insaturation(s) éthylénique(s) :

Il s'agit d'un groupe de polymères de type polyester présentant au moins 2 doubles liaisons éthyléniques, réparties de manière aléatoire dans la chaîne principale du polymère. Ces polyesters insaturés sont obtenus par polycondensation d'un mélange :

- de diacides carboxyliques aliphatiques linéaires ou ramifiés ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturations éthyléniques tels que les dimères des acides oléique ou linoléique décrits dans la demande EP-A-959 066 (paragraphe [0021]) commercialisés sous les dénominations Pripol® par la société Unichema ou Empol® par la société Henkel, tous ces diacides devant être exempts de doubles liaisons éthyléniques polymérisables,
- de diols aliphatiques linéaires ou ramifiés ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le le bisphénol A et le bisphénol B, et/ou de dimères diols issus de la réduction des dimères d'acides gras tels que définis précédemment, et
- d'un ou de plusieurs diacides carboxyliques ou leurs anhydrides comportant au moins une double liaison éthylénique polymérisable et ayant de 3 à 50 atomes de carbone, de préférence de 3 à 20 et mieux de 3 à 10 atomes de carbone, tels que l'acide maléique, l'acide fumarique ou l'acide itaconique.

b) les polyesters à groupes (méth)acrylate latéraux et/ou terminaux :

Il s'agit d'un groupe de polymères de type polyester obtenus par polycondensation d'un mélange :

- de diacides carboxyliques aliphatiques linéaires ou ramifiés ou cycloaliphatiques comportant notamment de 3 à 50 atomes de carbone, de préférence de 3 à 20 et mieux de 3 à 10 atomes de carbone, tels que l'acide adipique ou l'acide sébacique, de diacides carboxyliques aromatiques ayant notamment de 8 à 50 atomes de carbone, de préférence de 8 à 20 et mieux de 8 à 14 atomes de carbone, tels que les acides phtaliques, notamment l'acide téréphtalique, et/ou de diacides carboxyliques issus de dimères d'acides gras à insaturation éthyléniques tels que les dimères des acides oléique ou linoléique décrits dans la demande EP-A-959 066 (paragraphe [0021]) commercialisés sous les dénominations Pripol® par la société Unichema ou Empol® par la société Henkel, tous ces diacides devant être exempts de doubles liaisons éthyléniques polymérisables,
- de diols aliphatiques linéaires ou ramifiés ou cycloaliphatiques comportant notamment de 2 à 50 atomes de carbone, de préférence de 2 à 20 et mieux de 2 à 10 atomes de carbone, tels que l'éthylèneglycol, le diéthylèneglycol, le propylèneglycol, le 1,4-butanediol ou le cyclohexanediméthanol, de diols aromatiques ayant de 6 à 50 atomes de carbone, de préférence de 6 à 20 et mieux de 6 à 15 atomes de carbone tel que le bisphénol A et le bisphénol B, et
- d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.

Ces polyesters diffèrent de ceux décrits ci-dessus sous le point a) par le fait que les doubles liaisons éthyléniques ne sont pas situées dans la chaîne principale mais sur des groupes latéraux ou à l'extrémité des chaînes. Ces doubles liaisons éthyléniques sont celles des groupes (méth)acrylate présents dans le polymère.

De tels polyesters sont commercialisés par exemple par la société UCB sous les dénominations EBECRYL® (EBECRYL® 450 : masse molaire 1600, en moyenne 6 fonctions acrylate par molécule, EBECRYL® 652 : masse molaire 1500, en moyenne 6 fonctions acrylate par molécule, EBECRYL® 800 : masse molaire 780, en moyenne 4 fonctions acrylate par molécule, EBECRYL® 810 : masse molaire 1000, en moyenne 4 fonctions acrylate par molécule, EBECRYL® 50 000 : masse molaire 1500, en moyenne 6 fonctions acrylate par molécule).

c) les polyuréthannes et/ou polyurées à groupes (méth)acrylate, obtenus par polycondensation :

- de diisocyanates, triisocyanates et/ou polyisocyanates aliphatiques cycloaliphatiques et/ou aromatiques ayant notamment de 4 à 50, de préférence de 4 à 30 atomes de carbone, tels que l'hexaméthylènediisocyanate, l'isophoronediisocyanate, le toluènediisocyanate, le diphénylméthanediisocyanate ou les isocyanurates de formule :

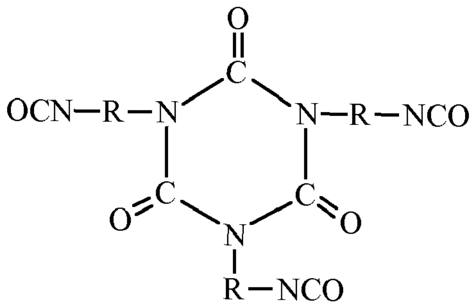

résultant de la trimérisation de 3 molécules de diisocyanates OCN-R-CNO, où R est un radical hydrocarboné linéaire, ramifié ou cyclique comportant de 2 à 30 atomes de carbone ;
- de polyols, notamment de diols, exempts d'insaturations éthyléniques polymérisables, tels que le 1,4-butanediol, l'éthylèneglycol ou le triméthylolpropane, et/ou de polyamines, notamment de diamines, aliphatiques, cycloaliphatiques et/ou aromatiques ayant notamment de 3 à 50 atomes de carbone, telles que l'éthylènediamine ou l'hexaméthylènediamine, et
- d'au moins un ester d'acide (méth)acrylique et d'un diol ou polyol ayant de 2 à 20 atomes de carbone, de préférence de 2 à 6 atomes de carbone, tels que le (méth)acrylate de 2-hydroxyéthyle, le (méth)acrylate de 2-hydroxypropyle et le méthacrylate de glycérol.

De tels polyuréthannes/polyurées à groupes acrylates sont commercialisés par exemple sous la dénomination SR 368 (tris(2-hydroxyéthyl)isocyanurate-triacrylate) ou CRAYNOR® 435 par la société CRAY VALLEY, ou sous la dénomination EBECRYL® par la société UCB (EBECRYL® 210 : masse molaire 1500, 2 fonctions acrylate par molécule, EBECRYL® 230 : masse molaire 5000, 2 fonctions acrylate par molécule, EBECRYL® 270 : masse

molaire 1500, 2 fonctions acrylate par molécule, EBECRYL® 8402 : masse molaire 1000, 2 fonctions acrylate par molécule, EBECRYL® 8804 : masse molaire 1300, 2 fonctions acrylate par molécule, EBECRYL® 220 : masse molaire 1000, 6 fonctions acrylate par molécule, EBECRYL® 2220 : masse molaire 1200, 6 fonctions acrylate par molécule, EBECRYL® 1290 : masse molaire 1000, 6 fonctions acrylate par molécule, EBECRYL® 800 : masse molaire 800, 6 fonctions acrylate par molécule).
On peut également citer les polyuréthannes aliphatiques diacrylate hydrosolubles commercialisés sous les dénominations EBECRYL® 2000, EBECRYL® 2001 et EBECRYL® 2002, et les polyuréthannes diacrylate en dispersion aqueuse commercialisés sous les dénominations commerciales IRR® 390, IRR® 400, IRR® 422 IRR® 424 par la société UCB.

d) les polyéthers à groupes (méth)acrylate obtenus par estérification, par l'acide (méth)acrylique, des groupes hydroxyle terminaux d'homopolymères ou de copolymères d'alkylèneglycols en $C_{1-4}$, tels que le polyéthylèneglycol, le polypropylèneglycol, les copolymères d'oxyde d'éthylène et d'oxyde de propylène ayant de préférence une masse moléculaire moyenne en poids inférieure à 10 000, le triméthylolpropane polyéthoxylé ou polypropoxylé.

Des polyoxyéthylènes-di(méth)acrylate de masse molaire appropriée sont commercialisés par exemple sous les dénominations SR 259, SR 344, SR 610, SR 210, SR 603 et SR 252 par la société CRAY VALLEY ou sous la dénomination EBECRYL® 11 par UCB. Des triacrylates de triméthylolpropane polyéthoxylé sont commercialisés par exemple sous les dénominations SR 454, SR 498, SR 502, SR 9035, SR 415 par la société CRAY VALLEY ou sous la dénomination EBECRYL® 160 par la société UCB. Des triacrylates de triméthylolpropane polypropoxylé sont commercialisés par exemple sous les dénominations SR 492 et SR 501 par la société CRAY VALLEY.

e) les époxyacrylates obtenus par réaction entre

- au moins un diépoxyde choisi par exemple parmi :

  (i) l'éther diglycidylique de bisphénol A,
  (ii) une résine diépoxy résultant de la réaction entre l'éther diglycidylique de bisphénol A et l'épichlorhydrine,
  (iii) une résine époxyester à extrémités $\alpha,\omega$-diépoxy résultant de la condensation d'un diacide carboxylique ayant de 3 à 50 atomes de carbone avec un excès stoechiométrique de (i) et/ou (ii),
  (iv) une résine époxyéther à extrémités $\alpha,\omega$-diépoxy résultant de la condensation d'un diol ayant de 3 à 50 atomes de carbone avec un excès stoechiométrique de (i) et/ou (ii),
  (v) les huiles naturelles ou synthétiques portant au moins 2 groupes époxyde, telles que l'huile de soja époxydée, l'huile de lin époxydée et l'huile de vernonia époxydée,
  (vi) un polycondensat phénol-formaldéhyde (résine Novolac®), dont les extrémités et/ou les groupes latéraux ont été époxydés,
  et

- un ou plusieurs acides carboxyliques ou polyacides carboxyliques comportant au moins une double liaison éthylénique en $\alpha,\beta$ du groupe carboxylique comme l'acide (méth)acrylique ou l'acide crotonique ou les esters d'acide (méth)acrylique et d'un diol ou polyol ayant de 2 à 20 atomes de carbone, de préférence de 2 à 6 atomes de carbone tels que le (méth)acrylate de 2-hydroxyéthyle.

De tels polymères sont commercialisés par exemple sous les dénominations SR 349, SR 601, CD 541, SR 602, SR 9036, SR 348, CD 540, SR 480, CD 9038 par le société CRAY VALLEY, sous les dénominations EBECRYL® 600 et EBECRYL® 609, EBECRYL® 150, EBECRYL® 860, EBECRYL® 3702 par la société UCB et sous les dénominations PHOTOMER® 3005 et PHOTOMER® 3082 par la société HENKEL.

f) les poly(méth)acrylates d'(alkyle en $C_{1-50}$), ledit alkyle étant linéaire, ramifié ou cyclique, comportant au moins deux fonctions à double liaison éthylénique portées par les chaînes hydrocarbonées latérales et/ou terminales.
De tels copolymères sont commercialisés par exemple sous les dénominations IRR® 375, OTA® 480 et EBECRYL® 2047 par la société UCB.

g) les polyoléfines telles que le polybutène, le polyisobutylène,

h) les perfluoropolyéthers à groupes acrylate obtenus par estérification, par exemple par l'acide (méth)acrylique,

de perfluoropolyéthers portant des groupes hydroxyle latéraux et/ou terminaux.
De tels perfluoropolyéthers $\alpha,\omega$-diols sont décrits notamment dans EP-A-1057849 et sont commercialisés par la société AUSIMONT sous la dénomination FOMBLIN® Z DIOL.

i) les dendrimères et polymères hyperramifiés portant des groupes terminaux (méth)acrylate ou (méth)acrylamide obtenus respectivement par estérification ou amidification de dendrimères et de polymères hyperramifiés à fonctions terminales hydroxyle ou amino, par de l'acide (méth)acrylique.
Les dendrimères (du grec dendron = arbre) sont des molécules polymères "arborescentes", c'est-à-dire très ramifiées inventées par D. A. Tomalia et son équipe au début des années 90 (Donald A. Tomalia et al., Angewandte Chemie, Int. Engl. Ed., vol. 29, n° 2, pages 138 - 175). Il s'agit de structures construites autour d'un motif central généralement polyvalent. Autour de ce motif central, sont enchaînés, selon une structure parfaitement déterminée, des motifs ramifiés d'allongement de chaîne donnant ainsi naissance à des macromolécules symétriques, monodispersées ayant une structure chimique et stéréochimique bien définie. Des dendrimères de type polyamidoamine sont commercialisés par exemple sous la dénomination STARBUST® par la société DENDRITECH.
Les polymères hyperramifiés sont des polycondensats, généralement de type polyester, polyamide ou polyéthylèneamine, obtenus à partir de monomères multifonctionnels, qui ont une structure arborescente similaire à celle des dendrimères mais beaucoup moins régulière que celle-ci (voir par exemple WO-A-93/17060 et WO 96/12754). La société PERSTORP commercialise sous la dénomination BOLTORN® des polyesters hyperramifiés. On trouvera sous la dénomination COMBURST® de la société DENDRITECH des polyéthylèneamines hyperramifiées. Des poly (esteramide) hyperramifiés à extrémités hydroxyle sont commercialisés par la société DSM sous la dénomination HYBRANE®.
Ces dendrimères et polymères hyperramifiés estérifiés ou amidifiés par l'acide acrylique et/ou méthacrylique se distinguent des polymères décrits sous les points a) à h) ci-dessus par le très grand nombre de doubles liaisons éthyléniques présentes. Cette fonctionnalité élevée, le plus souvent supérieure à 5, les rend particulièrement utiles en leur permettant de jouer un rôle de "noeud de réticulation", c'est-à-dire de site de réticulation multiple.

On peut donc utiliser ces polymères dendritiques et hyperramifiés en association avec un ou plusieurs des polymères et/ou oligomères a) à h) ci-dessus.

**Ia - Composés réactifs additionnels**

Selon un mode de réalisation, les compositions comprenant le composé X et/ou Y peut comprendre en outre un composé réactif additionnel tels que :

- les particules organiques ou minérales comprenant à leur surface au moins 2 groupements aliphatiques insaturés, on peut citer par exemple les silices traitées en surface par exemple par des composés siliconés à groupements vinyliques tels que par exemple la silice traitée cyclotetraméthyltetravinylsiloxane,
- des composés silazanes tels que l'hexaméthyldisilazane.

**1b - Catalyseur**

La réaction d'hydrosilylation se fait en présence d'un catalyseur qui peut être présent avec l'un ou l'autre des composés X ou Y ou être présent de manière isolée. Par exemple, ce catalyseur peut être présent dans la composition sous une forme encapsulée si les deux composés X et Y, dont il doit provoquer l'interaction, sont présents dans cette même composition sous une forme non encapsulée ou à l'inverse il peut y être présent sous une forme non encapsulée si au moins l'un des composés X et Y est présent dans la composition sous une forme encapsulée. Le catalyseur est de préférence à base de platine ou d'étain.
On peut citer par exemple les catalyseurs à base de platine déposé sur un support de gel de silice ou de poudre de charcoal (charbon), le chlorure de platine, les sels de platine et d'acides chloroplatiniques.
On utilise de préférence les acides chloroplatiniques sous forme hexahydrate ou anhydre, facilement dispersible dans les milieux organosiliconés.
On peut également citer les complexes de platine tels que ceux à base d'acide chloroplatinique hexahydrate et de divinyl tetraméthyldisiloxane.
Le catalyseur peut être présent en une teneur allant de 0,0001% à 20% en poids par rapport au poids total de la composition le comprenant.
Les composés X et/ou Y peuvent être associés à des inhibiteurs ou retardateurs de polymérisation, et plus particulièrement des inhibiteurs du catalyseur. De façon non limitative, on peut citer les polyméthylvinylsiloxanes cycliques, et en particulier le tetravinyl tétraméthyl cyclotetrasiloxane, les alcools acétyléniques, de préférence volatils, tels

que le méthylisobutynol.

La présence de sels ioniques, tels que l'acétate de sodium peut avoir une influence dans la vitesse de polymérisation des composés.

A titre d'exemple d'une combinaison de composés X et Y réagissant par hydrosilylation en présence d'un catalyseur, on peut citer les références suivantes proposée par la société Dow Corning : DC 7-9800 Soft Skin Adhesive Parts A & B, ainsi que la combinaison des mélanges A et B suivants préparés par Dow Corning :

**MELANGE A :**

| **Ingrédient (Nom INCI)** | **N°CAS** | **Teneurs (%)** | **Fonction** |
|---|---|---|---|
| Dimethyl Siloxane, Dimethylvinylsiloxy-terminaux | 68083-19-2 | 55-95 | Polymère |
| Silica Silylate | 68909-20-6 | 10-40 | Charge |
| 1,3-Diethenyl-1,1,3,3-Tetramethyldisiloxane complexes | 68478-92-2 | Trace | Catalyseur |
| Tetramethyldivinyldisiloxane | 2627-95-4 | 0.1-1 | Polymère |

MELANGE B :

| **Ingrédient (Nom INCI)** | **N°CAS** | **Teneurs (%)** | **Fonction** |
|---|---|---|---|
| Dimethyl Siloxane, Dimethylvinylsiloxy-terminaux | 68083-19-2 | 55-95 | Polymère |
| Silica Silylate | 68909-20-6 | 10-40 | Charge |
| Dimethyl, Methylhydrogen Siloxane, trimethylsiloxy-terminaux | 68037-59-2 | 1-10 | Polymère |

De façon avantageuse, les composés X et Y sont choisis parmi les composés siliconés susceptibles de réagir par hydrosilylation en présence d'un catalyseur ; en particulier le composé X est choisi parmi les polyorganosiloxanes comprenant des unités de formule (I) décrits ci-dessus et le composé Y est choisi parmi les organosiloxanes comprenant des unités alkylhydrogènosiloxanes de formule (III) décrits ci-dessus.

Selon un mode de réalisation particulier, le composé X est un polydiméthylsiloxane à groupements vinyliques terminaux, et le composé Y est un polyméthylhydrogénosiloxane.

**2/ Composés X et Y susceptibles de réagir par condensation**

Selon un mode de réalisation, l'invention concerne des particules de type coeur/écorce à coeur lipophile caractérisées en ce que leur écorce est formée au moins en partie d'un film polymérique siliconé qui est le produit de réaction *in situ* entre au moins un composé X et au moins un composé Y, l'un au moins des composés X et Y étant un polyorganosiloxane et ladite réaction étant de type condensation, le cas échéant réalisée en présence d'un catalyseur.

Selon ce mode de réalisation, les composés X et Y sont susceptibles de réagir par condensation, soit en présence d'eau (hydrolyse) par réaction de 2 composés porteurs de groupements alcoxysilanes, soit par condensation dite « directe » par réaction d'un composé porteur de groupement(s) alcoxysilane(s) et d'un composé porteur de groupement(s) silanol(s) ou par réaction de 2 composés porteurs de groupement(s) silanol(s).

Lorsque la condensation se fait en présence d'eau, celle-ci peut être en particulier l'humidité ambiante, l'eau résiduelle de la peau, des lèvres des cils et/ou des ongles, ou l'eau apportée par une source extérieure, par exemple par humidification préalable de la matière kératinique (par exemple par un brumisateur, des larmes naturelles ou artificielles).

Dans ce mode de réaction par condensation, les composés X et Y, identiques ou différents, peuvent donc être choisis parmi les composés siliconés dont la chaîne principale comprend au moins deux groupes alcoxysilane et/ou au moins deux groupes silanol (Si-OH), latéraux et/ou en bout de chaîne.

Selon un mode de réalisation, le composé X et/ou le composé Y est porteur d'au moins un groupe polaire, tel que décrit ci-dessus, susceptible de former au moins une liaison hydrogène avec les matières kératiniques.

Selon un mode de réalisation avantageux, les composés X et/ou Y sont choisis parmi les polyorganosiloxanes comprenant au moins deux groupes alcoxysilane. Par groupe « alcoxysilane », on entend un groupe comprenant au moins une partie -Si-OR, R étant un groupe alkyle comprenant de 1 à 6 atomes de carbone.

Les composés X et Y sont notamment choisis parmi les polyorganosiloxanes comprenant des groupes terminaux alcoxysilanes, plus spécifiquement ceux qui comprennent au moins 2 groupes alcoxysilanes terminaux, de préférence trialcoxysilanes terminaux.

Ces composés X et/ou Y comprennent de préférence de façon majoritaire des unités de formule :

$$R^9_sSiO_{(4-s)/2} \quad (IV)$$

dans laquelle les groupes $R^9$ représentent indépendamment les uns des autres un radical choisi parmi les groupes alkyle comprenant de 1 à 6 atomes de carbone, le phényle, les groupes fluoroalkyle, et s est égal à 0, 1, 2 ou 3. De préférence, les groupes $R^9$ représentent indépendamment les uns des autres un groupe alkyle comprenant de 1 à 6 atomes de carbone. Comme groupe alkyle, on peut citer notamment le méthyle, le propyle, le butyle, l'hexyle et leurs mélanges, de préférence le méthyle ou l'éthyle. Comme groupe fluoroalkyle, on peut citer le 3, 3, 3-trifluoropropyle.

Selon un mode de réalisation particulier, les composés X et Y, identiques ou différents, sont des polyorganosiloxanes comprenant des unités de formule :

$$(R^9_2SiO_2)_f - \quad (V)$$

dans laquelle $R^9$ est tel que décrit ci-dessus, de préférence $R^9$ est un radical méthyle, et f est tel que le polymère présente avantageusement une viscosité à 25°C allant de 0,5 à 3000 Pa.s, de préférence allant de 5 à 150 Pa.s ; par exemple f peut aller de 2 à 5000, de préférence de 3 à 3000, et préférentiellement de 5 à 1000.

Ces composés X et Y polyorganosiloxanes comprennent au moins 2 groupes trialcoxysilanes terminaux par molécule de polymère, lesdits groupes ayant la formule suivante

$$-ZSiR^1_x(OR)_{3-x}, \quad (VI)$$

dans laquelle :

les radicaux R représentent indépendamment un groupe méthyle, éthyle, n-propyle, isopropyle, n-butyle, sec-butyle, isobutyle, de préférence un groupe méthyle ou éthyle,

$R^1$ est un groupe méthyle ou éthyle,

x est égal à 0 ou 1, de préférence x est égal à 0 et

Z est choisi parmi : les groupes hydrocarbonés divalents ne comportant pas d'insaturation éthylénique et comprenant de 1 à 18 atomes de carbone, de préférence de 2 à 18 atomes de carbone (groupes alkylène), les combinaisons de radicaux hydrocarbonés divalents et de segments siloxanes de formule (IX) suivante :

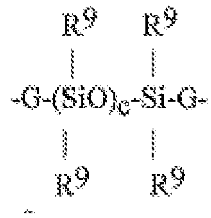

(IX)

$R^9$ étant tel que décrit plus haut, G est un radical hydrocarboné divalent ne comportant pas d'insaturation éthylénique et comprenant de 1 à 18 atomes de carbone, de préférence de 2 à 18 atomes de carbone et c est un entier allant de 1 à 6. Z et G peuvent être notamment choisis parmi les groupements alkylènes tels que le méthylène, l'éthylène, le propylène, le butylène, le pentylène, l'hexylène, les groupements arylène tels que le phénylène.

De préférence, Z est un groupe alkylène, et mieux éthylène.

Ces polymères peuvent présenter en moyenne au moins 1,2 groupements terminaux ou chaînes terminales trialcoxysilanes par molécule, et de préférence en moyenne au moins 1,5 groupements terminaux trialcoxysilanes par molécule. Ces polymères pouvant présenter au moins 1,2 groupements terminaux trialcoxysilanes par molécule, certains peuvent comprendre d'autre types de groupes terminaux tels que des groupements terminaux de formule $CH_2$=CH-

$SiR^9_2$- ou de formule $R^6_3$-Si-, dans laquelle $R^9$ est tel que défini plus haut et chaque groupe $R^6$ est indépendamment choisi parmi les groupes $R^9$ ou vinyle. On peut citer comme exemples de tels groupements terminaux les groupes triméthoxysilane, triéthoxysilane, vinyldiméthoxysilane et vinylméthylo xyphénylsilane.

De tels polymères sont notamment décrits dans les documents US 3 175 993, US 4 772 675, US 4 871 827, US 4 888 380, US 4 898 910, US 4 906 719 et US 4 962 174 dont le contenu est incorporé par référence à la présente demande.

On peut citer à titre de composé X et/ou Y en particulier les polyorganosiloxanes choisis parmi les polymères de formule :

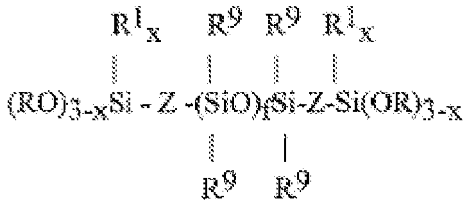

(VII)

dans laquelle R, $R^1$, $R^9$, Z, x et f sont tels que décrits plus haut.

Les composés X et/ou Y peuvent également comprendre un mélanges de polymère de formule (VII) ci-dessus avec des polymères de formule (VIII) suivante :

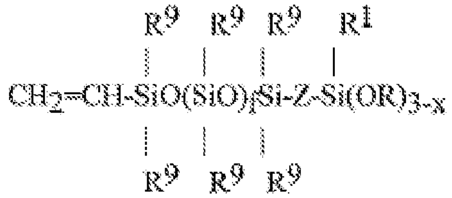

(VIII)

dans laquelle R, $R^1$, $R^9$, Z, x et f sont tels que décrits plus haut.

Lorsque le composé X et/ou Y polyorganosiloxanes à groupe(s) alcoxysilane(s) comprend un tel mélange, les différents polyorganosiloxanes sont présents en des teneurs telles que les chaînes organosilyles terminales représentent moins de 40%, de préférence moins de 25% en nombre des chaînes terminales.

Les composés X et/ou Y polyorganosiloxanes particulièrement préférés sont ceux de formule (VII) décrits ci-dessus. De tels composés X et/ou Y sont décrits par exemple dans le document WO 01/96450.

Comme indiqué plus haut, les composés X et Y peuvent être identiques ou différents.

En particulier, les composés X et Y peuvent représenter un mélange de polydiméthylsiloxanes à groupements méthoxysilanes.

Selon une variante, l'un des 2 composés réactifs X ou Y, est de nature siliconée et l'autre est de nature organique. Par exemple le composé X est choisi parmi les oligomères ou polymères organiques ou les oligomères ou polymères hybrides organique/silicone, lesdits polymères ou oligomères comprenant au moins deux groupements alcoxysilanes et Y est choisi parmi les composés siliconés tels que les polyorganosiloxanes décrits ci-dessus. En particulier, les oligomères ou polymères organiques sont choisis parmi les oligomères ou polymères vinyliques, (méth)acryliques, polyesters, polyamides, polyuréthanes et/ou polyurées, polyéthers, polyoléfines, perfluoropolyéthers, dendrimères et polymères hyper-ramifiés organiques, et leurs mélanges.

Selon un mode de réalisation, le composé X de nature organique ou de nature hybride organique/silicone est porteur d'au moins un groupe polaire, tel que décrit ci-dessus, susceptible de former au moins une liaison hydrogène avec la matière kératinique.

Les polymères organiques de nature vinylique ou (méth)acryliques, porteurs de groupes latéraux alcoxysilanes, pourront en particulier être obtenus par copolymérisation d'au moins un monomère organique vinylique ou (méth)acrylique avec un (méth)acryloxypropyltriméthoxysilane, un vinyl triméthoxysilane, un vinyltriéthoxysilane, un allyltriméthoxysilanes etc..

On peut citer par exemple les polymère (méth)acryliques décrits dans le document de KUSABE.M, Pitture e Verniei - European Coating ; 12-B, pages 43-49, 2005, et notamment les polyacrylates à groupes alcoxysilanes

référencés MAX de Kaneka ou ceux décrits dans la publication de PROBSTER, M, Adhesion-Kleben & Dichten, 2004, 481 (1-2), pages 12-14.

Les polymères organiques résultant d'une polycondensation ou d'une polyaddition, tel que les polyesters, polyamides, polyuréthanes et/ou polyurées, polyéthers, et porteurs de groupes alcoysilanes latéraux et/ou terminaux, pourront résulter par exemple de la réaction d'un prépolymère oligomère tel que décrit plus haut avec l'un des co-réactifs silanes suivant porteurs d'au moins un groupe alcoxysilane : aminopropyltriméthoxysilane, aminopropyltriéthoxysilane, aminoéthyl aminopropyl triméthoxysilane, glycidoxypropyltriméthoxysilane, glycidoxypropyltriéthoxysilane, époxycyclohéxyléthyltriméthoxysilane, mercaptopropyltriméthoxysilane.

Des exemples de polyéthers et de polyisobutylènes à groupes alcoxysilanes sont décrits dans la publication de KUSABE.M., Pitture e Verniei - European Coating ; 12-B, pages 43-49, 2005. Comme exemple de polyuréthanes à groupes alcoxysilanes terminaux, on peut citer ceux décrits dans le document PROBSTER, M., Adhesion-Kleben & Dichten, 2004, 481 (1-2), pages 12-14 ou encore ceux décrits dans le document LANDON, S., Pitture e Verniei vol. 73, N° 11, pages 18-24, 1997 ou dans le document HUANG, Mowo, Pitture e Verniei vol. 5, 2000, pages 61-67, on peut notamment citer les polyuréthanes à groupes alcoxysilanes de OSI-WITCO-GE.

A titre de composés X et/ou Y polyorganosiloxane, on peut citer les résines de type MQ ou MT portant elle-même des extrémités alcoxysilanes et/ou silanols comme par exemple les résines poly(isobutylsilsesquioxane) fonctionnalisées par des groupes silanols proposées sous la référence SST-S7C41 (3 groupes Si-OH) par la société Gelest.

**2a Composé réactif additionnel**

La réaction de polycondensation peut également être conduite en présence d'un composé réactif additionnel comprenant au moins deux groupes alcoxysilane ou silanol.

On peut citer par exemple :

- une ou des particules organiques ou minérales comprenant à leur surface des groupement alcoxysilanes et/ou silanols, par exemple des charges traitées en surface par de tels groupes.

**2b Catalyseur**

La réaction de condensation peut se faire en présence d'un catalyseur à base de métal qui peut être présent dans la phase organique de l'étape 1. Il peut être introduit via l'une ou l'autre des compositions comprenant X et/ou Y ou dans une composition séparée. Le catalyseur utile dans ce type de réaction est de préférence un catalyseur à base de titane.

On peut citer notamment les catalyseurs à base de tetraalcoxytitane de formule $Ti(OR^2)_y(OR^3)_{4-y}$, dans laquelle $R^2$ est choisi parmi les radicaux alkyle tertiaires tels que le tert butyle, le tert amyle et le 2,4-diméthyl-3-pentyle ; $R^3$ représente un radical alkyle comprenant de 1 à 6 atomes de carbone, de préférence un groupe méthyle, éthyle, n-propyle, isopropyle, n-butyle, sec-butyle, hexyle et y est un nombre allant de 3 à 4, mieux de 3,4 à 4.

Le catalyseur peut être présent dans la composition en une teneur allant 0,0001 % à 20 % en poids par rapport au poids total de la composition le contenant.

A titre d'exemple d'une combinaison de composés X et Y porteurs de groupements alcoxysilanes et réagissant par condensation, on peut citer la combinaison des mélanges A' et B' suivants préparés par la société Dow Corning :

**Mélange A' :**

| Ingrédient (Nom INCI) | N°CAS | Teneurs (%) | Fonction |
|---|---|---|---|
| Bis-Trimethoxysiloxyethyl Tetramethyldisilo xyethyl Dimethicone (1) | PMN87176 | 25-45 | Polymère |
| Silica Silylate | 68909-20-6 | 5-20 | Charge |
| Disiloxane | 107-46-0 | 30-70 | Solvant |

**Mélange B' :**

| Ingrédient (Nom INCI) | N°CAS | Teneurs (%) | Fonction |
|---|---|---|---|
| Disiloxane | 107-46-0 | 80-99 | Solvant |
| Tetra T Butyl Titanate | - | 1-20 | Catalyseur |

Il est à noter que les composés X et Y, identiques, sont réunis dans le mélange A' (cf. (1))

**3/ Réticulation en présence de peroxyde :**

Selon un mode de réalisation, l'invention concerne des particules de type coeur/écorce à coeur lipophile caractérisées en ce que leur écorce est formée au moins en partie d'un film polymérique siliconé qui est le produit de réaction *in situ* entre au moins un composé X et au moins un composé Y, l'un au moins des composés X et Y étant un polyorganosiloxane et ladite réaction étant de type réticulation réalisée en présence d'un péroxyde.

Cette réaction se fait de préférence par chauffage à une température supérieure ou égale à 50 °C, de préférence supérieure ou égale à 80 °C, allant jusqu'à 120 °C.

Les composés X et Y, identiques ou différents, comprennent dans ce cas au moins deux groupements latéraux $-CH_3$ et/ou au moins deux chaînes latérales portant un groupement $-CH_3$.

Les composés X et Y sont de préférence des polyorganosiloxanes et peuvent être choisis par exemple parmi les polydiméthylsiloxanes linéaires non volatiles de haut poids moléculaire, ayant un degré de polymérisation supérieur à 6 présentant au moins deux groupements latéraux $-CH_3$ reliés à l'atome de silicium et/ou au moins deux chaînes latérales portant un groupement $-CH_3$. On peut citer par exemple les polymères décrits dans le Catalogue « Reactive Silicones » de la société Gelest Inc., Edition 2004, page 6, et notamment les copolymères (aussi appelés gommes) de vinylméthylsiloxane-diméthylsiloxane de poids moléculaires allant de 500 000 à 900 000 et notamment de viscosité supérieure à 2 000 000 cSt.

A titre de peroxydes utilisables dans le cadre de l'invention, on peut citer le peroxyde de benzoyle, le peroxyde de 2,4-dichlorobenzoyle et leurs mélanges.

Selon un mode de réalisation, la réaction d'hydrosilylation en présence d'un catalyseur, ou la réaction de condensation le cas échéant en présence d'un catalyseur, ou bien encore la réaction de réticulation en présence d'un peroxyde, entre les composés X et Y est accélérée par un apport de chaleur en élevant par exemple la température du système entre 25 °C et 180 °C.

D'une façon générale, quel que soit le type de réaction par laquelle les composés X et Y réagissent ensemble, le pourcentage molaire de X par rapport à l'ensemble des composés X et Y, c'est-à-dire le ratio X/(X+Y) x 100, peut varier de 5 % à 95 %, de préférence de 10 % à 90 %, mieux encore de 20 % à 80 %.

De même, le pourcentage molaire de Y par rapport à l'ensemble des composés X et Y, c'est-à-dire le ratio Y/(X+Y) x 100, peut varier de 5 % à 95 %, de préférence de 10 % à 90 %, mieux encore de 20 % à 80 %.

Le composé X peut présenter une masse moléculaire moyenne en poids (Mw) allant de 150 à 1 000 000, de préférence de 200 à 800 000, de préférence encore de 200 à 250 000.

Le composé Y peut présenter une masse moléculaire moyenne en poids (Mw) allant de 200 à 1 000 000, de préférence de 300 à 800 000, de préférence encore de 500 à 250 000.

Le composé X peut représenter de 0,1 % à 95 % en poids par rapport au poids total de la composition le contenant de préférence de 1 % à 90 % et mieux de 5 % à 80 %.

Le composé Y peut représenter de 0,1 % à 95 % en poids par rapport au poids total de la composition le contenant de préférence de 1 % à 90 % et mieux de 5 % à 80 %.

Le ratio entre les composés X et Y peut être varié de manière à moduler la vitesse de réaction et donc la vitesse de formation du film ou encore de manière à adapter les propriétés de l'écorce formée (par exemple ses propriétés adhésives) selon l'application recherchée.

En particulier, les composés X et Y peuvent être présents en un ratio X/Y molaire allant de 0,05 à 20 et mieux de 0,1 à 10.

Les composés X et Y peuvent avantageusement être associés à au moins une charge. Ainsi, les particules selon l'invention ou compositions les contenant peuvent par exemple comprendre au moins une charge choisie parmi la silice ou la silice traitée en surface.

**COEUR DES PARTICULES SELON L'INVENTION**

Le coeur des particules selon l'invention est formé d'au moins un composé lipophile. Il peut notamment comprendre au moins une huile, un actif sous forme d'huile ou un actif solubilisé dans une phase huileuse.

Au sens de la présente invention, le terme « huile » désigne toutes les substances huileuses, liquides à 40 °C, naturelles, végétales ou animales, ou synthétiques ayant ou non une ou plusieurs activités biologiques avérées et insolubles dans l'eau (moins de 2 % en poids à température ambiante).

Les huiles susceptibles d'être mises en oeuvre au niveau du coeur des particules selon l'invention peuvent notamment être choisies parmi celles présentes ci-après dans la définition du milieu physiologiquement acceptable.

Le coeur peut également contenir au moins un agent actif lipophile différent d'une huile.

Peuvent ainsi également être encapsulés une ou plusieurs matières actives liposolubles qui seront solubilisées

dans une huile solvante.

Selon cette variante, c'est le mélange d'au moins deux composantes voire plus qui est encapsulé.

A titre d'exemple de ces principes actifs liposolubles, on peut citer le D α tocophérol, le DL α tocophérol, l'acétate de D α tocophérol, l'acétate DL α tocophérol, le palmitate d'ascorbyle, les glycérides de vitamine F, les vitamines D, la vitamine $D_2$, la vitamine $D_3$, le rétinol, les esters de rétinol, le palmitate de rétinol, le propionate de rétinol, les carotènes, dont le β carotène, le D-panthénol, le farnesol, l'acétate de farnesyle, l'acide salicylique et ses dérivés comme l'acide n-octanoyl-5 salicylique, les alkylesters d'α hydroxyacides tels que l'acide citrique, l'acide lactique, l'acide glycolique, l'acide asiatique, l'acide madécassique, l'asiaticoside, l'extrait total de *centella asiatica,* l'acide β glycyrrhétinique, le α bisabolol, les céramides comme le 2 oleoylamino-1,3 octadécane, le phytanetriol, les phospholipides d'origine marine riches en acides gras essentiels polyinsaturés, l'éthoxyquine, l'extrait de romarin, l'extrait de mélisse, la quercetine, l'extrait de microalgues séchées (algoxan red de Algatec), l'huile essentielle de bergamotte, l'octylmethoxycinnamate (Parsol MCX - Givaudan - Roure), le butylméthoxydibenzoylméthane (Parsol 1789 - Givaudan - Roure), l'octyl triazone (Uvinul T150 - BASF), et le di tertiobutyl-3,5 hydroxy-4 benzylidène 3 camphre.

Par ailleurs, peuvent également être encapsulées, toutes molécules à effet thérapeutique utilisées dans le domaine pharmaceutique dans la mesure où elles présentent un caractère huileux et/ou une solubilité suffisante dans l'huile considérée. Il peut ainsi s'agir d'anti-inflammatoires, stéroïdiens ou non, d'antifongiques, d'antibactériens, d'antibiotiques, d'antimitotiques, d'anesthésiques, d'analgésiques, d'antiseptiques, d'antiviraux.

Mais il peut également s'agir de composés liposolubles actifs dans d'autres domaines, comme par exemple des pesticides ou des herbicides, ou d'un mélange des agents actifs précités.

Bien entendu, le taux en huile(s) et le cas échéant en matière(s) active(s) liposoluble(s) (huile ou huile+actif) solubilisé dans le solvant, est fonction de la solubilité de ceux-ci.

## PROCEDE DE PREPARATION DES PARTICULES SELON L'INVENTION

Comme précisé précédemment les particules selon l'invention peuvent être obtenues selon des technologies conventionnelles reposant soit sur le principe de l'émulsification d'une phase organique faiblement miscible à l'eau dans une phase aqueuse soit sur celui du basculement de solvant consistant à mettre en présence une phase organique miscible à l'eau avec une phase aqueuse. A l'issue de ces deux protocoles, les particules formées peuvent être récupérées par évaporation du solvant organique.

Ces deux techniques sont en particulier respectivement décrites dans les documents FR 2 864 900 et EP 274 961.

Lors de la mise en contact des deux phases, selon l'une ou l'autre des deux technologies précitées, il y a formation de gouttelettes et réaction *in situ* entre les composés X et Y, voire le ou les composés réactifs additionnels si présents, et donc formation des particules attendues.

En ce qui concerne la première variante reposant sur la formation d'une émulsion phase organique dans eau, elle met en oeuvre un outil d'émulsification classique. La taille des gouttes de l'émulsion formée par mise en présence de la phase organique avec la phase aqueuse, peut être affinée par divers moyens connus comme l'homogénéisation haute pression ou les ultra sons. Enfin le solvant organique est évaporé. Lors de cette évaporation, les composés X et Y se concentrent dans la phase organique et la réaction a lieu entre ces deux composés en présence le cas échéant d'un catalyseur ou d'un peroxyde si nécessaire à leur interaction pour former l'écorce des particules.

Quant à la seconde variante, elle implique la solubilisation du composé lipophile et des composés X et Y destinés à former le polymère devant constituer l'enveloppe de la particule, dans un solvant organique en présence le cas échéant d'un catalyseur ou d'un peroxyde si nécessaire à leur interaction. La formation des particules est initiée par addition de la phase aqueuse, sous agitation, à la phase organique. Une émulsion spontanée se forme lorsque la phase aqueuse diffuse dans la phase organique. Les composés X et Y réagissent alors ensemble pour former le film polymérique siliconé qui constitue l'écorce des particules. Le ou les solvants organiques sont ensuite évaporés.

## A PHASE ORGANIQUE

Quelle que soit la variante considérée, la phase organique, est formée d'un ou plusieurs solvants organiques miscibles entre eux et dont le point ébullition est inférieur à celui de l'eau et donc généralement inférieur à 100 °C à la pression atmosphérique.

Dans le cas de la première variante, la phase organique est faiblement miscible à l'eau.

Au sens de l'invention, l'expression faiblement miscible à l'eau signifie que le solvant organique considéré manifeste une miscibilité dans l'eau inférieure à 10 % en poids à température ambiante.

A titre d'exemple de solvant convenant à l'invention, on peut en particulier citer l'acétate d'éthyle, l'acétate de butyle, le dichlorométhane, le cyclohexane, l'heptane, le chloro-1-butane, le chloroforme, le formate d'éthyle et leurs mélanges.

En revanche, dans le cas de la seconde variante, la phase organique est miscible à l'eau. A titre d'exemple de solvant convenant à cette deuxième variante, on peut citer l'acétone ainsi que les alcools inférieurs tels que l'éthanol, l'isopropanol, le méthanol.

Quelque soit la variante considérée, la phase organique contient au moins les composés X et Y, sous formes solubilisées et un ou plusieurs composés lipophiles à encapsuler.

Outre ces composés, la phase organique peut comprendre un composé réactif additionnel tel que décrit précédemment, un catalyseur ou un peroxyde si nécessaire à la réaction entre les composés X et Y, et éventuellement un composé apte à former une phase lamellaire en surface des particules attendues.

Dans tous les cas, les composés X et Y qui sont dilués dans la phase organique, ne réagissent pas entre eux.

**B <u>PHASE AQUEUSE</u>**

Pour sa part, la phase aqueuse contient avantageusement au moins un tensioactif pour, d'une part, faciliter la formation de l'émulsion se formant lors de la mise en contact des deux phases et, d'autre part, la stabiliser.

Cet agent tensioactif ou ce mélange de tensioactifs est généralement présent de 0,05 à 25 % et notamment de 1 à 20 % en poids par rapport au poids du milieu solvant organique à disperser. Sa valeur HLB (Balance Hydrophile-Lipophile) est généralement ajustée pour être favorable à la formation des émulsions de type huile dans eau.

Conviennent notamment à l'invention, les agents tensioactifs non ioniques suivants :

- les alkylester ou éther de glycérol ou de polyglygérol constitués de 1 à 10 « motif » glycérol et d'au moins une chaîne alkyle (acide pour les esters et alcool pour les éthers) ayant de 12 à 22 atomes de carbone. Elle peut être saturée ou insaturée et ramifiée ou non. A titre d'exemple, on peut citer le Nikkol DGMS® (monostéarate de diglycérol), le Nikkol decaglyn 2IS® (di isostéarate de décaglycérol), l'hexadécyléther de triglycérol,
- les esters mixtes d'acides gras ou d'alcool gras, d'acide carboxylique et de glycéryl choisis par exemple parmi les esters mixtes d'acide gras ou d'alcool gras en $C_8$-$C_{22}$ et d'alpha-hydroxyacide et/ou d'acide succinique avec la glycérine et leurs mélanges. A titre d'exemple, on peut citer l'ester mixte de glycérine et du mélange d'acides citrique, lactique, linoléique et oléique (nom CTFA : Glyceryl citrate/lactate/linoleate/oleate) commercialisé par la société Hüls sous la dénomination Imwitor 375® ; l'ester mixte d'acide succinique et d'alcool isostéarylique avec la glycérine (nom CTFA : Isostéaryl diglycéryl succinate) commercialisé par la société Hüls sous la dénomination Imwitor 780 K®; l'ester mixte d'acide citrique et d'acide stéarique avec la glycérine (nom CTFA : Glycéryl stéarate citrate) commercialisé par la société Hüls sous la dénomination Imwitor 370® ; l'ester mixte d'acide lactique et d'acide stéarique avec la glycérine (nom CTFA : Glyceryl stearate lactate) commercialisé par la société Danisco sous la dénomination Lactodan B30® ou Rylo LA30®,
- les éthers gras éthoxylés ou esters gras éthoxylés comprenant de 2 à 50 unités d'oxyde d'éthylène et au moins une chaîne alkyle ( acide pour les esters et alcool pour les éthers) ayant de 12 à 22 atomes de carbone. Elle peut être saturée ou insaturée et ramifiée ou non. A titre d'exemple, on citera la série des Brij® ( alcools gras éthoxylés) commercialisée par la société Uniqema, la série des Myrj® (stéarates éthoxylés) commercialisée par la société Uniqema et l'isostéarate de PEG400 également commercialisé par Uniqema,
- les esters gras de sorbitan, oxyéthylénés ou non. Ils comprennent au moins un motif sorbitan et dans le cas où ils sont oxyéthylénés, de 2 à 50 motifs d'oxyde d'éthylène, et au moins une chaîne alkyle (acide gras) ayant de 12 à 22 atomes de carbone. Elle peut être saturée ou insaturée et ramifiée ou non. A titre d'exemple, on citera les séries des Span® (esters de sorbitan) et des Tween® (esters de sorbitan oxyéthylénés) commercialisées par Uniqema,
- les esters gras de sucre ou éthers gras de sucre. Le tensioactif utilisé est par exemple choisi parmi les esters d'acide gras en $C_8$ à $C_{22}$ de sucrose, maltose, glucose et fructose, les esters d'acide gras en $C_{14}$ à $C_{22}$ et de méthylglucose, les alkylpolyglucosides et leurs mélanges. La ou les chaînes alkyles peuvent être saturée ou insaturée et ramifiée ou non.

  Les acides gras en $C_8$-$C_{22}$ ou en $C_{14}$-$C_{22}$ formant le motif gras des esters utilisables dans la composition de l'invention comportent au moins une chaîne alkyle linéaire saturée ou non saturée, comportant respectivement de 8 à 22 ou de 14 à 22 atomes de carbone. Le motif gras des esters peut être notamment choisi parmi les stéarates, béhénates, arachidonates, palmitates, myristates, laurates, caprates et leurs mélanges. On utilise notamment des stéarates.

  On peut citer, à titre d'exemple d'esters ou de mélanges d'esters d'acide gras et de sucrose, de maltose, de glucose ou de fructose, le monostéarate de sucrose, le distéarate de sucrose, le tristéarate de sucrose et leurs mélanges, tels que les produits commercialisés par la société Croda sous la dénomination Crodesta® F50, F70, F110, F160 ayant respectivement un HLB (Balance Hydrophile Lipophile) de 5, 7, 11 et 16 ; et à titre d'exemple d'esters ou de mélanges d'esters d'acide gras et de méthylglucose, le distéarate de méthyl glucose et de polyglycérol-3, vendu par la société Goldschmidt sous la dénomination de Tego-care 450®. On peut citer aussi les monoesters de glucose ou de maltose tels que l'o-hexadécanoyle-6-D-glucoside de méthyle et l'o-hexadécanoyle-6-D-maltoside.

Les éthers d'alcool gras et de sucre, utilisables comme tensioactifs peuvent être choisis notamment dans le groupe comprenant les éthers ou mélanges d'éthers d'alcool gras en $C_8$-$C_{22}$ et de glucose, de maltose, de sucrose ou de fructose et les éthers ou mélanges d'éthers d'alcool gras en $C_{14}$-$C_{22}$ et de méthylglucose. Ce sont notamment des alkylpolyglucosides.

Les alcools gras en $C_8$-$C_{22}$ ou en $C_{14}$-$C_{22}$ formant le motif gras des éthers utilisables selon l'invention comportent une chaîne alkyle linéaire saturée ou non saturée, comportant respectivement de 8 à 22 ou de 14 à 22 atomes de carbone. Le motif gras des éthers peut être notamment choisi parmi les motifs décyle, cétyle, béhényle, arachidyle, stéaryle, palmityle, myristyle, lauryle, capryle, hexadécanoyle et leurs mélanges tels que cétéaryle.

A titre d'exemple d'éthers d'alcool gras et de sucre, on peut citer les alkylpolyglucosides tels que le décyl glucoside et le lauryl glucoside commercialisés par exemple par la société Henkel sous les dénominations respectives Plantaren 2000® et Plantaren 1200®, le cétostéaryl glucoside éventuellement en mélange avec l'alcool cétostéarylique, commercialisé par exemple sous la dénomination Montanov 68® par la société Seppic, sous la dénomination Tegocare CG90® par la société Goldschmidt et sous la dénomination Emulgade KE3302® par la société Henkel, ainsi que l'arachidyl glucoside, par exemple sous la forme du mélange d'alcools arachidique et béhénique et d'arachidyl glucoside commercialisé sous la dénomination Montanov 202® par la société Seppic,

- les copolymères blocs d'oxyde d'éthylène et d'oxyde de propylène. Les copolymères blocs d'oxyde d'éthylène et d'oxyde de propylène, utilisables comme tensioactifs dans les compositions selon l'invention peuvent être choisis notamment parmi les copolymères blocs de formule (A) :

$$HO(C_2H_4O)_x(C_3H_6O)_y(C_2H_4O)_zH \quad (A)$$

dans laquelle x, y et z sont des nombres entiers tels que x+z peut aller de 2 à 280 et peut aller de 14 à 100. Ces polymères sont notamment commercialisés sous le nom de Pluronic®
ou Lutrol® par BASF, Symperonic® par Uniqema,

- les lécithines de soja ou d'oeuf hydrogénée ou non, enrichies en phosphatidylcholine ou non,
- les tensioactifs siliconé comportant au moins une chaîne oxyéthylénée et ou oxypropylénée. On peut citer, à titre d'exemple, ceux décrits dans les brevets US A 5364633 et US A 5411744 et en particulier un composé de formule (I) :

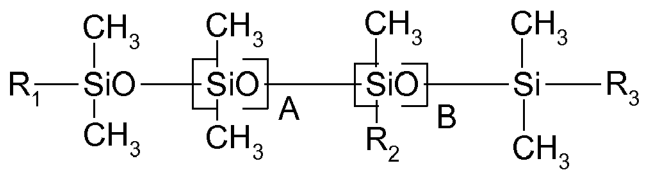

(I)

dans laquelle :

$R_1$, $R_2$, $R_3$, indépendamment les uns des autres, représentent un radical alkyle en $C_1$-$C_6$ ou un radical $-(CH_2)_x - (OCH_2CH_2)_y - (OCH_2CH_2CH_2)_z - OR_4$, au moins un radical $R_1$, $R_2$ ou $R_3$ n'étant pas un radical alkyle ; $R_4$ étant un hydrogène, un radical alkyle ou un radical acyle ;
A est un nombre entier allant de 0 à 200 ;
B est un nombre entier allant de 0 à 50 ; à la condition que A et B ne soient pas égaux à zéro en même temps ;
x est un nombre entier allant de 1 à 6 ;
y est un nombre entier allant de 1 à 30 ;
z est un nombre entier allant de 0 à 5.

Selon un mode de réalisation particulier, dans le composé de formule (I), le radical alkyle est un radical méthyle, x est un nombre entier allant de 2 à 6 et y est un nombre entier allant de 4 à 30.

On peut citer, à titre d'exemple de tensioactifs siliconés de formule (I), les composés de formule (II) :

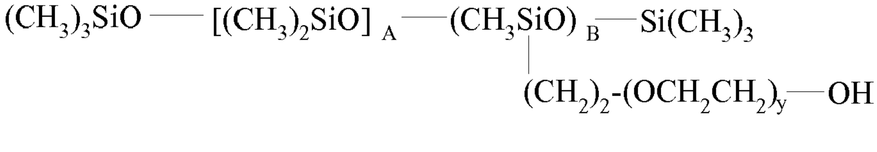

(II)

dans laquelle A est un nombre entier allant de 20 à 105, B est un nombre entier allant de 2 à 10 et y est un nombre

entier allant de 10 à 20.

On peut également citer à titre d'exemple de tensioactifs siliconés de formule (I), les composés de formule (III) :

$$HO - (CH_2CH_2O)_y\text{-}(CH_2)_3 - [(CH_3)_2SiO]_{A'} \text{-}(CH_2)_3\text{-}(OCH_2CH_2)_y - OH \text{ (III)}$$

dans laquelle A' et y sont des nombres entiers allant de 10 à 20.

On peut utiliser comme composés de l'invention ceux vendus par la société Dow Corning sous les dénominations DC 5329, DC 7439-146, DC 2-5695 et Q4-3667. Les composés DC 5329, DC 7439-146, DC 2-5695 sont des composés de formule (II) où respectivement A est 22, B est 2 et y est 12 ; A est 103, B est 10 et y est 12 ; A est 27, B est 3 et y est 12. Le composé Q4-3667 est un composé de formule (III) où A est 15 et y est 13. Mais aussi :

- les citrates d'alkyléther,
- les alkényl succinates alkoxylés,
- les alkényl succinates de glucose alkoxylés,
- les alkényl succinates de méthylglucose alkoxylés.

Ces tensioactifs peuvent être utilisés seuls ou en association. Leur taux, par rapport à la phase huileuse encapsulée (après évaporation du solvant) peut être compris entre 0,1 et 30 % en poids.

Afin de parfaire la stabilité de cette émulsion, voire de réduire un peu plus la taille des gouttes du milieu solvant organique, il peut être avantageux d'ajouter de 0,01 à 5 % en poids par rapport au poids total de la dispersion d'au moins un tensioactif ionique, soluble dans l'eau et ayant une HLB supérieure à 11. Ce type de tensioactif ionique génère semble-t-il une charge électrique à la surface des nanocapsules et favorise ainsi la manifestation de répulsions électrostatiques entres elles. Ce ou ces tensioactifs ioniques, anioniques ou cationiques peuvent être choisis parmi :

- les lipides amphiphiles anioniques comme :

- les sels alcalins du dicétyl- et du dimyristyl-phosphate,
- les sels alcalins du cholestérol sulfate,
- les sels alcalins du cholestérol phosphate,
- les lipoaminoacides et leurs sels tels que les acylglutamates mono- et disodiques comme le sel disodique de l'acide N-stéaroyl L-glutamique commercialisé sous la dénomination Amisoft HS21P par la société AJINOMOTO,
- les sels de sodium de l'acide phosphatidique,
- les phospholipides,
- les dérivés alkylsulfoniques notamment de formule :

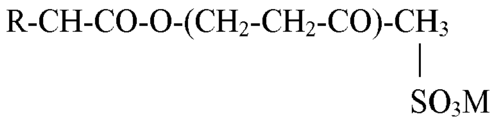

dans laquelle R représente des radicaux alkyle en $C_{16}$-$C_{22}$, en particulier les radicaux $C_{16}H_{33}$ et $C_{18}H_{37}$ pris en mélange ou séparément et M est un métal alcalin ou alcalino-terreux tel que le sodium,

- les lipides amphiphiles cationiques de type sels d'ammonium quaternaire, amines grasses et leurs sels comme par exemple :

- les sels d'ammonium quaternaires de formule générale (IV) suivante :

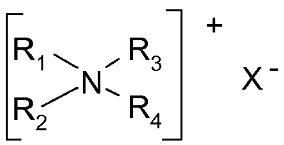 (IV)

dans laquelle les radicaux $R_1$, $R_2$, $R_3$ et $R_4$ peuvent être identiques ou différents, représentent un radical aliphatique,

linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène($C_2$-$C_6$), alkylamide, alkyl($C_{12}$-$C_{22}$)amido alkyle($C_2$-$C_6$), alkyl($C_{12}$-$C_{22}$)acétate, hydroxyalkyle, comportant environ de 1 à 30 atomes de carbone ; X est un anion choisi dans le groupe des halogénures, phosphates, acétates, lactates, alkyl($C_2$-$C_6$) sulfates, alkyl-ou-alkylarylsulfonates. Comme sels d'ammonium quaternaire de formule (IV), on préfère, d'une part, les chlorures de tétraalkylammonium comme par exemple les chlorures de dialkyldiméthylammonium ou d'alkyltriméthylammonium, dans lesquels le radical alkyle comporte environ de 12 à 22 atomes de carbone, en particulier les chlorures de béhényltriméthylammonium, de distéaryldiméthyl-ammonium, de cétyltriméthyl-ammonium, de benzyl diméthyl stéaryl-ammonium ou encore, d'autre part, le chlorure de stéaramidopropyldiméthyl (acétate de myristyle) ammonium vendu sous la dénomination «CERAPHYL 70®» par la société VAN DYK,

- les sels d'ammonium quaternaire de l'imidazolinium, comme par exemple ceux de formule (V) suivante :

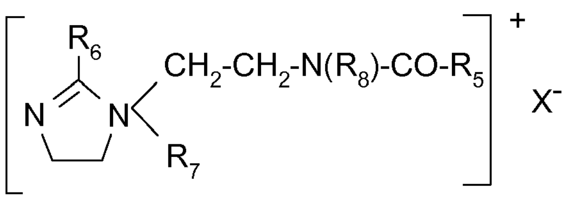

(V)

dans laquelle $R_5$ représente un radical alcényle ou alkyle comportant de 8 à 30 atomes de carbone, par exemple dérivé des acides gras du suif ; $R_6$ représente un atome d'hydrogène, un radical alkyle comportant de 1 à 4 atomes de carbone ou un radical alcényle ou alkyle comportant de 8 à 30 atomes de carbone ; $R_7$ représente un radical alkyle comportant de 1 à 4 atomes de carbone ; $R_8$ représente un atome d'hydrogène, un radical alkyle comportant de 1 à 4 atomes de carbone ; X est un anion choisi dans le groupe des halogénures, phosphates, acétates, lactates, alkylsulfates, alkyl-ou-alkylarylsulfonates. De préférence, $R_5$ et $R_6$ désignent un mélange de radicaux alcényle ou alkyle comportant de 12 à 21 atomes de carbone, par exemple dérivés des acides gras du suif, $R_7$ désigne un radical méthyle, $R_8$ désigne l'hydrogène. Un tel produit est par exemple vendu sous la dénomination «REWOQUAT W 75» par la société REWO,

- les sels de diammonium quaternaire de formule (VI) suivante :

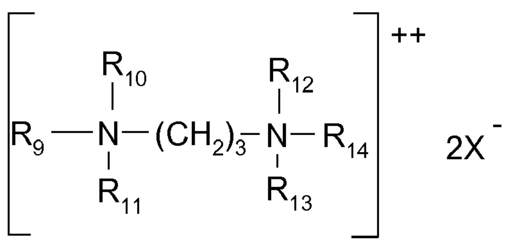

(VI)

dans laquelle $R_9$ désigne un radical aliphatique comportant environ de 16 à 30 atomes de carbone ; $R_{10}$, $R_{11}$, $R_{12}$, $R_{13}$, et $R_{14}$ sont choisis parmi l'hydrogène ou un radical alkyle comportant de 1 à 4 atomes de carbone; et X est un anion choisi dans le groupe des halogénures, acétates, phosphates, nitrates et méthylsulfates. De tels sels de diammonium quaternaire comprennent notamment le dichlorure de propane suif diammonium.

Le taux de tensioactif ionique lorsqu'il est associé au(x) tensioactif(s) non ionique(s) présent(s) dans le milieu aqueux est généralement ajusté de manière à représenter de 2 à 100 % en poids du poids de celui-ci.

**C <u>COMPOSE APTE A FORMER UNE PHASE LAMELLAIRE</u>**

Il est souvent souhaitable ou nécessaire de pourvoir les particules, par exemple les microparticules ou les nanocapsules d'un enrobage dit « lamellaire ». Il s'agit d'une structure organisée en un ou plusieurs feuillet(s) lipidique (s) constitué(s) chacun d'une bicouche de molécules amphiphiles semblable à celle des membranes biologiques. L'enveloppe polymérique des particules, par exemple les microparticules ou lesnanocapsules selon l'invention peut être ainsi entourée d'un enrobage lamellaire ayant une structure organisée en un ou plusieurs feuillet(s) constitué(s) chacun

d'une double couche de molécules amphiphiles constituant un agent d'enrobage. Cet enrobage, outre sa fonction d'ajustement de la taille des particules, par exemple les microparticules ou les nanocapsules, améliore l'étanchéité des particules, par exemple les microparticules ou les nanocapsules vis-à-vis d'une fuite de l'actif vers une autre phase lipidique de la composition.

On entend par « phase lamellaire » (phase D selon EKWALL), une phase cristal liquide à symétrie plane, comprenant plusieurs bicouches d'amphiphile disposées en parallèle et séparées par un milieu liquide qui est généralement de l'eau.

Une définition plus précise de cette dénomination est donnée dans Ekwall (1968), Adv. Liq. Cryst. (Brown G.H., Ed.), Chap. 1, 14. Cette phase possède une texture caractéristique au microscope en lumière polarisée dont on pourra trouver une description plus précise dans Roservear (1968), JSCC, 19, 581. et dans Lachampt et Vila (1969), Revue Française des Corps Gras, n° 2, 87-111.

L'enrobage lamellaire est obtenu avec des agents tensioactifs à caractère hydrophobe, solubles dans la phase organique utilisée dans le procédé décrit ci-dessus et qui sont capables, en présence d'eau, de former les doubles couches lipidiques décrites ci-dessus. Dans le procédé d'encapsulation, utilisé par les inventeurs, le tensioactif d'enrobage est dissous dans la phase organique contenant les composés X et Y et la phase lipidique.

On peut citer à titre d'exemple de tels tensioactifs d'enrobage, les phospholipides tels que la lécithine comme décrit dans le document EP-A-447 318 ; certains polycondensats d'oxyde d'éthylène et d'oxyde de propylène, comme les produits vendus sous la dénomination PLURONIC® par la société BASF tels que PLURONIC® L121 ou sous la dénomination SYNPERONIC® par la société ICI; ou les agents tensioactifs siliconés (silicones comportant au moins une chaîne oxyéthylénée et/ou oxypropylénée) capables de former des structures lamellaires, tels que ceux décrits dans les documents US-A-5,364,633 et US-A-5,411,744 utilisés dans la demande de brevet FR-A-2,742,677, par exemple ceux vendus par la société DOW CORNING sous les dénominations DC 5329, DC 7439-146, DC 2-5695 et Q4-3667 ; et leurs mélanges.

Les particules, par exemple les nanocapsules ou microcapsules, obtenues peuvent être introduites dans tous les types de formulation galénique à condition qu'ils assurent leur stabilité, tels que les gels, les émulsions huile/eau, eau/huile, multiples (E/H/E, H/E/H), les sérums, les lotions.... On pourra introduire de 0,5 à 60 % de suspension de particules selon l'invention dans la formulation finale.

Elles peuvent notamment mises en oeuvre à titre de véhicule d'agent actif lipophile dans des compositions cosmétiques ou thérapeutiques.

Comme précisé précédemment, une composition conforme à l'invention comprend un milieu physiologiquement acceptable.

Par « milieu physiologiquement acceptable », on entend désigner un milieu convenant particulièrement à l'application d'une composition selon l'invention sur la peau, les lèvres, les cils, les sourcils ou les ongles. Le milieu physiologiquement acceptable est généralement adapté à la nature du support sur lequel doit être appliquée la composition, ainsi qu'à l'aspect sous lequel la composition est destinée à être conditionnée.

Le milieu physiologiquement acceptable peut comprendre une phase aqueuse comprenant, essentiellement d'eau.

Elle peut également comprendre un mélange d'eau et de solvant miscible à l'eau (miscibilité dans l'eau supérieure à 50 % en poids à 25 °C) comme un, ou un mélange de monoalcool(s) inférieur(s) ayant de 1 à 5 atomes de carbone tel(s) que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, les aldéhydes en $C_2$-$C_4$ et leurs mélanges.

Le milieu physiologiquement acceptable peut également comprendre une phase grasse liquide, comprenant une ou plusieurs huiles volatiles ou non, et/ou une phase grasse solide comprenant une ou plusieurs cires et/ou composés pâteux, et leur mélange.

Par phase grasse liquide, au sens de la demande, on entend une phase grasse liquide à température ambiante (25°C) et pression atmosphérique (760 mm de Hg), composée d'un ou plusieurs corps gras non aqueux liquides à température ambiante, appelés aussi huiles ou solvants organiques.

L'huile peut être choisie parmi les huiles volatiles et/ou les huile non volatiles, et leurs mélanges.

La ou les huiles peuvent être présentes en une teneur allant de 1 % à 90 % en poids, de préférence de 5 % à 50 % en poids par rapport au poids total de la composition.

Ces huiles peuvent être des huiles hydrocarbonées, des huiles siliconées, des huiles fluorées, ou leurs mélanges.

On entend par « huile hydrocarbonée », une huile contenant principalement des atomes d'hydrogène et de carbone et éventuellement des atomes d'oxygène, d'azote, de soufre, de phosphore. Les huiles hydrocarbonées peuvent être choisies parmi les huiles hydrocarbonées ayant de 8 à 16 atomes de carbones, et notamment les alcanes ramifiés en $C_8$-$C_{16}$ comme les isoalcanes en $C_8$-$C_{16}$ d'origine pétrolière (appelées aussi isoparaffines) comme l'isododécane (encore appelé 2,2,4,4,6-pentaméthylheptane), l'isodécane, l'isohexadécane, et par exemple les huiles vendues sous

les noms commerciaux d'Isopars® ou de Permetyls®, les esters ramifiés en $C_8$-$C_{16}$ le néopentanoate d'iso-hexyle, et leurs mélanges.

Comme huile hydrocarbonée on peut également citer :

- les huiles hydrocarbonées d'origine végétale telles que les triesters d'acides gras et de glycérol dont les acides gras peuvent avoir des longueurs de chaînes variées de C4 à C24, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel ;
- les éthers de synthèse ayant de 10 à 40 atomes de carbone;
- les huiles hydrocarbonées apolaires comme le squalène, les hydrocarbures linéaires ou ramifiés tels que les huiles de paraffine, de vaseline et de naphtalène, le polyisobutène hydrogéné ou partiellement hydrogéné, l'isoeicosane, le squalane, les copolymères décène/butène, les copolymères polybutène/polyisobutène notamment l'Indopol L-14, les polydécènes tel que le PURESYN 10, et leurs mélanges;
- les esters de synthèse comme les huiles de formule $R_1COOR_2$ dans laquelle $R_1$ représente le reste d'un acide gras linéaire ou ramifié comportant de 1 à 40 atomes de carbone et $R_2$ représente une chaîne hydrocarbonée notamment ramifiée contenant de 1 à 40 atomes de carbone à condition que $R_1 + R_2$ soit $\geq 10$, comme par exemple l'huile de Purcellin (octanoate de cétostéaryle), le myristate d'isopropyle, le palmitate d'isopropyle, le benzoate d'alcool en $C_{12}$ à $C_{15}$, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
- et leurs mélanges.

Comme huile de silicone utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

Les huiles de silicone peuvent également être :

- les polydiméthylsiloxanes (PDMS),
- les polydiméthylsiloxanes comportant des groupements alkyle ou alcoxy, pendant et/ou en bout de chaîne siliconée, groupements ayant chacun de 3 à 40 atomes de carbone,
- les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates,
- et leurs mélanges.

La composition peut comprendre de 0,1 à 80 % en poids, de préférence de 1 à 60 % en poids, mieux de 5 à 50 % en poids et encore mieux de 14 à 40 % en poids d'huile(s) non volatile(s) par rapport à son poids total.

Les compositions selon l'invention peuvent également comprendre au moins un corps gras solides à température ambiante notamment choisi parmi les cires, les corps gras pâteux et leurs mélanges. Ces corps gras peuvent être d'origine animale, végétale, minérale ou synthétique.

Les compositions peuvent comprendre au moins une matière colorante choisie par exemple parmi les pigments, les nacres, les colorants, les matériaux à effet et leurs mélanges.

Par « colorants », il faut comprendre des composés, généralement organiques, solubles dans au moins une huile ou dans une phase hydroalcoolique.

Par pigments, il faut comprendre des particules blanches ou colorées, minérales ou organiques, insolubles dans un milieu aqueux, destinées à colorer et/ou opacifier le film résultant.

Par « nacres » ou pigments nacrés, il faut comprendre des particules colorées de toute forme, irisées ou non, notamment produites par certains mollusques dans leur coquille ou bien synthétisées et qui présentent un effet de

couleur par interférence optique.

Le pigment peut aussi être une laque. Par laque, on entend les colorants insolubilisés adsorbés sur des particules insolubles, l'ensemble ainsi obtenu restant insoluble lors de l'utilisation.

Les compositions cosmétiques selon l'invention peuvent également comprendre au moins une charge, notamment en une teneur allant de 0,01 % à 50 % en poids, de préférence allant de 0,01 % à 30 % en poids par rapport au poids total de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, la silice traitée en surface par un agent hydrophobe, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la Société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les compositions selon l'invention peuvent également contenir des ingrédients couramment utilisés en cosmétique, tels que les vitamines, les épaississants, les agents filmogènes, les gélifiants, les oligo-éléments, les adoucissants, les séquestrants, les parfums, les agents alcalinisants ou acidifiants, les conservateurs, les filtres solaires, les tensioactifs, les anti-oxydants, les fibres, les actifs de soin ou leurs mélanges.

Les compositions cosmétiques selon l'invention peuvent être des compositions de maquillage ou de soin des matières kératiniques, en particulier de la peau, des lèvres, des cils, des sourcils ou des ongles.

Les compositions de soin peuvent se présenter sous la forme d'une composition de de protection, de traitement ou de soin pour le visage, pour les mains, pour les pieds, pour les grands plis anatomiques ou pour le corps (par exemple crèmes de jour, crème de nuit, crème démaquillante, composition anti-solaire, lait corporels de protection ou de soin, laits après-solaire, lotion, gel ou mousse pour le soin de la peau, composition de bronzage artificiel); une composition après-rasage.

Il peut également s'agir de compositions plus particulièrement dédiées au maquillage comme un fond de teint, un produit anti-cerne, un baume à lèvres, un brillant à lèvres, un mascara, ou encore un produit de maquillage du corps ou de coloration de la peau.

Les exemples et figures soumis ci-après sont présents à titre illustratif et non limitatif de l'invention.

**Figure** :

La figure 1 rend compte des résultats d'un test de fuite réalisé avec les nanocapsules conformes à l'invention de l'exemple 2 en comparaison avec les nanocapsules témoins de l'exemple 3.

Dans les exemples de compositions décrites ci-après, on utilise, à titre de composés X et Y, la combinaison des mélanges A et B suivants préparés par la société Dow Corning :

**MELANGE A :**

| Ingrédient (Nom INCI) | N°CAS | Teneurs(%) | Fonction |
|---|---|---|---|
| Dimethyl Siloxane, Dimethylvinylsiloxy-terminaux | 68083-19-2 | 55-95 | Polymère |
| Silica Silylate | 68909-20-6 | 10-40 | Charge |
| 1,3-Diethenyl-1,1,3,3-Tetramethyldisiloxane complexes | 68478-92-2 | Trace | Catalyseur |
| Tetramethyldivinyldisiloxane | 2627-95-4 | 0.1-1 | Polymère |

**MELANGE B :**

| Ingrédient (Nom INCI) | N°CAS | Teneurs(%) | Fonction |
|---|---|---|---|
| Dimethyl Siloxane, Dimethylvinylsiloxy-terminaux | 68083-19-2 | 55-95 | Polymère |
| Silica Silylate | 68909-20-6 | 10-40 | Charge |
| Dimethyl, Methylhydrogen Siloxane, trimethylsiloxy-terminaux | 68037-59-2 | 1-10 | Polymère |

**Exemple 1 :**

Nanocapsules d'acétate de vitamine E conformes à l'invention

Phase organique :

| | |
|---|---|
| Acétate de tocophérol | 15g |
| Mélange A | 1g |
| Mélange B | 1g |
| Dichlorométhane | 66,7ml |

Phase aqueuse :

| | |
|---|---|
| Acide N Stéaroyl L glutamique disodique (Amisoft HS21P d'Ajinomoto) | 0,5g |
| Eau distillée | 82,5g |

Les constituants de la phase aqueuse sont introduits dans un bécher, sous agitation à 40°C. Puis cette phase aqueuse est refroidie à température ambiante.

Dans un autre bécher, les constituants de la phase organique sont dissous, sous agitation, à température ambiante.

Une émulsion est réalisée en versant la phase organique sur la phase aqueuse et en agitant à l'aide d'un agitateur de type rotor-stator comme un ultra turrax par exemple.

La taille des gouttes est ensuite affinée aux ultrasons.

Le dichlorométhane et une partie de l'eau sont évaporés à l'aide d'un évaporateur rotatif jusqu'à obtenir une masse finale de 100g.

On obtient une suspension de nanocapsules ayant un diamètre moyen de 256nm, mesuré par diffusion dynamique de la lumière sur un appareil BI90+ de chez Brookhaven Instruments.

**Exemple 2 :**

Nanocapsules de triglycérides caprylique/ caprique et contenant un marqueur conformes à l'invention

Phase organique :

| | |
|---|---|
| triglycérides caprylique/ caprique (MYRITOL 318 de chez Cognis) | 15g |
| Mélange A | 1,5g |
| Mélange B | 1,5g |
| 2,5-thiophenediylbis(5-tert-butyl-1,3-benzoxazole) (Uvitex OB de chez CIBA) | 0,15g |

(suite)

| | |
|---|---|
| Dichlorométhane | 66,7mL |

Phase aqueuse :

| | |
|---|---|
| Acide N Stéaroyl L glutamique disodique (Amisoft HS21P d'Ajinomoto) | 0,5g |
| Eau distillée | 81,35g |

On obtient une suspension de nanocapsules ayant un diamètre moyen de 262nm, mesuré par diffusion dynamique de la lumière sur un appareil BI90+ de chez Brookhaven Instruments.

**Exemple 3 :**

Nanocapsules de triglycérides caprylique/caprique et contenant un marqueur, non-conformes à l'invention car à base de polymère siliconé préformé

Phase organique :

| | |
|---|---|
| triglycérides caprylique/caprique (MYRITOL 318 de chez Cognis) | 2,5g |
| Polyméthylsilsesquioxane (Belsil PMS MK de chez Wacker) | 0,5g |
| Diméthicone copolyol (DC SH 3773 M de chez Dow Corning) | 0,5g |
| 2,5-thiophenediylbis(5-tert-butyl-1,3-benzoxazole) (Uvitex OB de chez CIBA) | 0,025g |
| Acétone | 100ml |

Phase aqueuse :

| | |
|---|---|
| condensat d'oxyde d'éthylène et d'oxyde de propylène et d'oxyde d'éthylène (128 OE/54 OP/128 OE) (synperonic PE/F108 de chez Uniquema) | 0,25g |
| Eau distillée | 200g |

Les différents constituants de la phase organique sont dissous dans un bécher, sous agitation, à température ambiante. Dans un autre bécher, les constituants de la phase aqueuse sont dissous, sous agitation, à température ambiante.

On verse la phase organique dans la phase aqueuse en maintenant l'agitation.

Puis on évapore l'acétone et une partie de l'eau à l'aide d'un évaporateur rotatif jusqu'à l'obtention d'un volume final de 50mL.

On obtient une suspension de nanocapsules ayant un diamètre moyen de 294nm, mesuré par diffusion dynamique de la lumière sur un appareil BI90+ de chez Brookhaven Instruments.

**Exemple 4 :**

Comparaison des nanocapsules des exemples 2 et 3 en terme de fuite du marqueur encapsulé

La suspension de nanocapsules obtenues dans l'exemple 2 est diluée 3 fois avec une solution aqueuse à 0.5% d'acide N Stéaroyl L glutamique disodique (Amisoft HS21P d'Ajinomoto). On obtient ainsi une suspension de nanocapsules dont la composition est la suivante :

| | |
|---|---|
| triglycérides caprylique/caprique (MYRITOL 318 de chez Cognis) | 5 |
| Mélange A | 0,5 |
| Mélange B | 0,5 |
| 2,5-thiophenediylbis(5-tert-butyl-1,3-benzoxazole) (Uvitex OB de chez CIBA) | 0,05g |
| Acide N Stéaroyl L glutamique disodique (Amisoft HS21P d'Ajinomoto) | 0,5g |
| Eau distillée | 93,45g |

Ces nanocapsules sont comparées à celles obtenues dans l'exemple 3 qui contiennent également 5 % d'huile encapsulée, 0,05 % de marqueur (uvitex OB) selon le test de fuite suivant :

On ajoute dans un flacon 40 g de suspension de nanocapsules et 40 g de capric/caprilic triglycérides qui sera appelé « phase huileuse extractante ».
On agite ces deux phases afin de former une émulsion grossière de la phase huileuse extractante dans la solution de nanocapsules.

A un temps T, on prélève 5mL du mélange. On centrifuge ces 5 mL à 50 000 rpm pendant 10 min afin de séparer la phase huileuse extractante de la suspension de nanocapsules.
Il a été au préalable vérifié que cette centrifugation ne détériore pas les nanocapsules et ne provoque donc pas de fuite supplémentaire.
On prélève la phase huileuse extractante surnageante et on la dilue dans l'acétone.
On mesure l'absorbance de la solution obtenue à 392 nm ce qui permet d'obtenir la concentration en uvitex OB ayant transféré du coeur des nanocapsules vers la phase huileuse extractante au temps T.
Ce test est réalisé en parallèle pour les deux suspensions de nanocapsules.
Les résultats sont illustrés en figure 1.
On constate que la fuite est plus faible pour les nanocapsules dont l'écorce est formée par la réaction des composés A et B considérés selon l'invention que pour un polymère de type polymethylsilsesquioxane. Après 11 jours, la fuite est de 14 % contre 27 %. La fuite est pratiquement divisée par un facteur 2.

## Revendications

1. Particule de type coeur/écorce à coeur lipophile **caractérisée en ce que** son écorce est formée au moins en partie d'un film polymérique siliconé qui est le produit de réaction *in situ* entre au moins un composé X et au moins un composé Y, l'un au moins des composés X et Y étant un polyorganosiloxane et ladite réaction étant de type hydrosilylation réalisée en présence d'un catalyseur, condensation le cas échéant réalisée en présence d'un catalyseur, ou réticulation réalisée en présence d'un peroxyde.

2. Particule selon la revendication 1 dont le coeur comprend au moins une huile et/ou un agent actif cosmétique ou thérapeutique lipophile.

3. Particule selon la revendication 1 ou 2 possédant un diamètre particulaire moyen variant de 50nm à 1μm.

4. Particule selon l'une quelconque des revendications précédentes dont l'écorce est en outre revêtue au moins en partie d'une phase lamellaire.

5. Particule selon l'une quelconque des revendications précédentes, **caractérisé en ce que** les composés X et Y sont susceptibles de réagir par hydrosilylation en présence d'un catalyseur.

6. Particule selon la revendication 5, dans laquelle le composé X est choisi parmi les composés siliconés comprenant au moins deux groupements aliphatiques insaturés.

7. Particule selon la revendication 6, dans laquelle le composé X est un polyorganosiloxane comprenant une chaîne principale siliconée dont les groupements aliphatiques insaturés sont pendants à la chaîne principale (groupe latéral)

ou situés aux extrémités de la chaîne principale du composé (groupe terminal).

**8.** Particule selon la revendication 7, dans laquelle le composé X est porteur d'au moins un groupe polaire.

**9.** Particule selon l'une des revendications 5 à 8 dans laquelle le composé X est choisi parmi les polyorganosiloxanes comprenant au moins deux groupements aliphatiques insaturés liés chacun à un atome de silicium.

**10.** Particule selon l'une des revendications 5 à 9 dans laquelle le composé X est choisi parmi les polyorganosiloxanes comprenant des unités siloxanes de formule :

$$R_m R' SiO_{\frac{(3-m)}{2}} \quad (I)$$

dans laquelle :

- R représente un groupe hydrocarboné monovalent, linéaire ou cyclique, comprenant de 1 à 30 atomes de carbone,
- m est égal à 1 ou 2 et
- R' représente :
- un groupement hydrocarboné aliphatique insaturé comprenant de 2 à 10, de préférence de 3 à 5 atomes de carbone ou
- un groupement hydrocarboné cyclique insaturé comprenant de 5 à 8 atomes de carbone.

**11.** Particule selon la revendication précédente dans laquelle le polyorganosiloxane de formule (I) est tel que R' représente un groupe vinyle ou un groupe -R"-CH=CHR"' dans lequel R" est une chaine hydrocarbonée aliphatique divalente, comprenant de 1 à 8 atomes de carbone, liée à l'atome de silicium et R"' est un atome d'hydrogène ou un radical alkyle comprenant de 1 à 4 atomes de carbone, de préférence un atome d'hydrogène.

**12.** Particule selon la revendication 10 ou 11 dans laquelle R représente un radical alkyle comprenant de 1 à 10 atomes de carbone ou encore un groupement phényle, et est de préférence un groupe méthyle, et R' est un groupe vinyle.

**13.** Particule selon l'une des revendications 5 à 12, dans laquelle les polyorganosiloxanes comprennent en outre des unités de formule

$$R_n SiO_{\frac{(4-n)}{2}} \quad (II)$$

dans laquelle R représente un groupe hydrocarboné monovalent, linéaire ou cyclique, comprenant de 1 à 30 atomes de carbone, et n est égal à 1, 2 ou 3.

**14.** Particule selon la revendication 5, dans laquelle le composé X est choisi parmi les oligomères ou polymères organiques, les oligomères ou polymères hybrides organique/silicone, lesdits oligomères ou polymères portant au moins 2 groupements aliphatiques insaturés réactifs.

**15.** Particule selon l'une des revendications 5 à 14, dans laquelle le composé Y comprend au moins deux groupes Si-H libres.

**16.** Particule selon l'une des revendications 5 à 15, dans laquelle le composé Y est choisi parmi les polyorganosiloxanes comprenant au moins une unité alkylhydrogènosiloxane de formule suivante :

$$R_pHSiO_{\left(\frac{3-p}{2}\right)} \qquad (III)$$

dans laquelle :

R représente un groupe hydrocarboné monovalent, linéaire ou cyclique, comprenant de 1 à 30 atomes de carbone, ou un groupe phényle et p est égal à 1 ou 2.

**17.** Particule selon la revendication précédente, dans laquelle le composé Y est tel que les radicaux R représentent un groupement alkyle en $C_1$-$C_{10}$, de préférence un groupement méthyle.

**18.** Particule selon l'une des revendications 15 à 17, dans laquelle Y est un polyorganosiloxane comprenant au moins deux unités alkylhydrogènosiloxane de formule $-(H_3C)(H)Si-O-$ et comprenant éventuellement des unités $-(H_3C)_2SiO-$.

**19.** Particule selon l'une des revendications 5 à 18, dérivant d'une hydrosilylation réalisée en présence d'un catalyseur à base de platine ou d'étain.

**20.** Particule selon la revendication précédente, dans laquelle le catalyseur représente de 0,0001 % à 20 % en poids par rapport à son poids total.

**21.** Particule selon l'une des quelconque des revendications 5 à 15, dans laquelle le composé X est un polydiméthylsiloxane à groupements vinyliques terminaux et le composé Y est un polyméthylhydrogénosiloxane.

**22.** Particule selon l'une des revendications 1 à 4 dans laquelle le composé X et le composé Y sont susceptibles de réagir par condensation.

**23.** Particule selon la revendication 22 dans laquelle les composés X et/ou Y, identiques ou diffférents, sont des composés siliconés dont la chaîne principale comprend au moins deux groupes alcoxysilane et/ou au moins deux groupes silanol (Si-OH), latéraux et/ou en bout de chaîne.

**24.** Particule selon la revendication 22 ou 23, dans laquelle les composés X et/ou Y, identiques ou différents comprennent de façon majoritaire des unités de formule

$$R^9_sSiO_{(4-s)/2}, \qquad (IV)$$

dans laquelle les groupes $R^9$ représentent indépendamment les uns des autres un radical choisi parmi les groupes alkyles comprenant de 1 à 6 atomes de carbone, le phényle, les groupes fluoro alkyles, et s est égal à 0, 1,2 ou 3.

**25.** Particule selon la revendication 24, dans laquelle les composés X et/ou Y, identiques ou différents sont des polyorganosiloxanes comprenant des unités de formule :

$$(R^9_2SiO_2)_f - \qquad (V)$$

dans laquelle $R^9$ est tel que défini dans la revendication 24, et f va de 2 à 5000.

**26.** Particule selon l'une des revendications 22 à 25, dans laquelle les composés X et/ou Y sont des polyorganosiloxanes comprenant au moins 2 groupes trialcoxysilane terminaux par molécule de polymère, lesdits groupes ayant la formule suivante :

$$-ZSiR^1_x(OR)_{3-x}, \quad (VI)$$

dans laquelle :

- les radicaux R représentent indépendamment un groupe méthyle, éthyle, n-propyle, isopropyle, n-butyle, sec-butyle, isobutyle,
- $R^1$ est un groupe méthyle ou éthyle,
- x est égal à 0 ou 1, de préférence x est égal à 0 et
- Z est choisi parmi : les groupes hydrocarbonés divalents ne comportant pas d'insaturation éthylénique et comprenant de 1 à 18 atomes de carbone, de préférence de 2 à 18 atomes de carbone, les combinaisons de radicaux hydrocarbonés divalents et de segments siloxanes de formule (IX) :

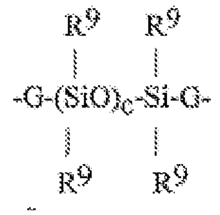

- $R^9$ étant tel que défini dans la revendication 24, G est un radical hydrocarboné divalent ne comportant pas d'insaturation éthylénique et comprenant de 1 à 18 atomes de carbone, de préférence de 2 à 18 atomes de carbone et c est un entier allant de 1 à 6.

**27.** Particule selon l'une des revendications 22 à 26, dans laquelle les composés X et/ou Y sont des polyorganosiloxanes sont choisis parmi les polymères de formule :

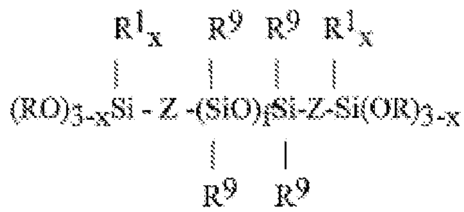

(VII)

dans laquelle R, $R^1$, $R^9$, Z, x et f sont tels que définis dans la revendication 26.

**28.** Particule selon la revendication 22, dans laquelle le composé X est choisi parmi les oligomères ou polymères organiques ou les oligomères et polymères hybrides organique/silicone, lesdits polymères ou oligomères comprenant au moins deux groupements alcoxysilanes et le composé Y est choisi parmi les polyorganosiloxanes.

**29.** Particule selon l'une des revendications 22 à 28, dérivant d'une polycondensation en présence d'un catalyseur à base de titane.

**30.** Particule selon la revendication 29, dans laquelle le catalyseur est présent en une teneur allant de 0,0001 % à 20 % en poids par rapport à son poids total.

**31.** Particule selon l'une quelconque des revendications 22 à 30 dans laquelle les composés X et Y représentent un mélange de polydiméthylsiloxanes à groupements méthoxysilanes.

**32.** Particule selon l'une des revendications 1 à 4, dans laquelle les composés X et Y sont susceptibles de réagir par réticulation en présence de peroxyde.

**33.** Particule selon l'une quelconque des revendications précédentes dans laquelle le composé X est porteur d'au moins un groupe polaire susceptible de former une liaison hydrogène avec les matières kératiniques.

**34.** Particule selon l'une des revendications précédentes, dans laquelle le composé X présente une masse moléculaire moyenne en poids (Mw) allant de 150 à 1 000 000, de préférence de 200 à 800 000, de préférence encore de 200 à 250 000.

**35.** Particule selon l'une des revendications précédentes, dans laquelle le composé Y présente une masse moléculaire en poids (Mw) allant de 200 à 1 000 000, de préférence de 300 à 800 000, de préférence encore de 500 à 250 000.

**36.** Particule selon l'une des revendications précédentes, dans laquelle les composés X et Y sont présents dans les particules en un ratio molaire X/Y allant de 0,05 à 20 et mieux de 0,1 à 10.

**37.** Particule selon l'une des revendications précédentes dont le coeur comprend au moins une huile.

**38.** Particule selon l'une quelconque des revendications précédentes, dans laquelle le coeur contient au moins un agent actif lipophile différent d'une huile.

**39.** Particule selon la revendication précédente, dans laquelle ledit agent actif est choisi parmi le D $\alpha$ tocophérol, le DL $\alpha$ tocophérol, l'acétate de D $\alpha$ tocophérol, l'acétate DL $\alpha$tocophérol, le palmitate d'ascorbyle, les glycérides de vitamine F, les vitamines D, la vitamine $D_2$, la vitamine $D_3$, rétinol, les esters de rétinol, le palmitate de rétinol, le propionate de rétinol, les carotènes dont le $\beta$ carotène, le D panthénol, le farnesol, l'acétate de farnesyle, l'acide salicylique et ses dérivés comme l'acide n-octanoyl-5 salicylique, les alkylesters d'$\alpha$ hydroxyacides tels que l'acide citrique, l'acide lactique, l'acide glycolique, l'acide asiatique, l'acide madécassique, l'asiaticoside, l'extrait total de *centella asiatica,* l'acide $\beta$ glycyrrhétinique, l'$\alpha$ bisabolol, les céramides comme le 2 oleoylamino-1,3 octadécane, le phytanetriol, les phospholipides d'origine marine riches en acides gras essentiels polyinsaturés, l'éthoxyquine, l'extrait de romarin, l'extrait de mélisse, le quercetine, l'extrait de microalgues séchées, l'huile essentielle de bergamotte, l'octylmethoxycinnamate, le butylméthoxydibenzoylméthane, l'octyl triazone, le di tertiobutyl-3,5 hydroxy-4 benzylidène 3 camphre, les antibiotiques, les antifongiques, les anesthésiques, les analgésiques, les antiseptiques, les antiviraux, les pesticides, les herbicides, les anti-inflammatoires, les antibactériens, les antimitotiques, et leurs mélanges.

**40.** Dispersion aqueuse de particules selon l'une quelconque des revendications précédentes.

**41.** Procédé de préparation de particules de type coeur/écorce dont le coeur est lipophile et l'écorce est formée au moins en partie d'un film polymérique siliconé comprenant au moins les étapes consistant à

i. solubiliser dans au moins un solvant organique volatil, au moins un composé lipophile, un composé X et un composé Y, avec au moins l'un des composés X et Y étant un organopolysiloxane et les composés X et Y étant dans des conditions non propices à leur interaction et,
ii. mettre en présence la phase organique de l'étape 1 avec une phase aqueuse contenant le cas échéant au moins un tensio-actif, dans des conditions propices à la formation de gouttelettes lipophiles et à la réaction des composés X et Y selon une réaction de type hydrosilylation en présence d'un catalyseur, condensation ou réticulation en présence d'un peroxyde pour former un film polymérique siliconé en surface desdite gouttelettes.

**42.** Procédé selon la revendication précédente dans lequel lesdites particules formées sont isolées par évaporation du ou des solvants organiques.

**43.** Procédé selon l'une des revendications 41 à 42 dans lequel le solvant organique est faiblement miscible à l'eau et l'étape 2 correspond à une émulsification de la phase organique de l'étape 1 au sein de la phase aqueuse à l'état de gouttelettes en surface desquelles les composés X et Y interagissent pour former le film polymérique constituant l'écorce desdites particules.

**44.** Procédé selon l'une des revendications 41 à 43, dans lequel le solvant organique est miscible à l'eau et l'étape 2 correspond à un basculement de solvant au cours duquel la diffusion de la phase aqueuse au sein de la phase organique provoque la formation de gouttelettes en surface desquelles les composés X et Y interagissent pour produire le film polymérique formant l'écorce desdites particules.

**45.** Procédé selon l'une des revendications 41 à 44, dans lequel les composés X et Y sont tels que définis en revendications 5 à 36.

**46.** Procédé selon l'une des revendications 41 à 45, dans lequel la phase organique de l'étape 1 contient un agent lipophile tel que défini en revendications 37 à 39.

**47.** Procédé selon l'une des revendications 41 à 46, dans lequel la phase organique de l'étape 1 comprend en outre un catalyseur.

**48.** Procédé selon l'une des revendications 41 à 47, dans lequel la phase organique de l'étape 1 comprend en outre un composé apte à former une phase lamellaire en surface desdites particules.

**49.** Composition cosmétique et/ou thérapeutique contenant dans un milieu physiologiquement acceptable au moins une particule selon l'une quelconque des revendications 1 à 39.

**50.** Composition selon la revendication précédente dans laquelle ladite particule contient au moins un agent lipophile choisi parmi le D $\alpha$ tocophérol, le DL $\alpha$ tocophérol, l'acétate de D $\alpha$ tocophérol, l'acétate DL $\alpha$tocophérol, le palmitate d'ascorbyle, les glycérides de vitamine F, les vitamines D, la vitamine $D_2$, la vitamine $D_3$, rétinol, les esters de rétinol, le palmitate de rétinol, le propionate de rétinol, les carotènes dont le $\beta$ carotène, le D panthénol, le farnesol, l'acétate de farnesyle, l'acide salicylique et ses dérivés comme l'acide n-octanoyl-5 salicylique, les alkylesters d'$\alpha$ hydroxyacides tels que l'acide citrique, l'acide lactique, l'acide glycolique, l'acide asiatique, l'acide madécassique, l'asiaticoside, l'extrait total de *centella asiatica,* l'acide $\beta$ glycyrrhétinique, l'$\alpha$ bisabolol, les céramides comme le 2 oleoylamino-1,3 octadécane, le phytanetriol, les phospholipides d'origine marine riches en acides gras essentiels polyinsaturés, l'éthoxyquine, l'extrait de romarin, l'extrait de mélisse, le quercetine, l'extrait de microalgues séchées, l'huile essentielle de bergamotte, l'octylmethoxycinnamate, le butylméthoxydibenzoylméthane, l'octyl triazone, le di tertiobutyl-3,5 hydroxy-4 benzylidène 3 camphre.

**51.** Composition selon l'une des revendications 49 ou 50, **caractérisée en ce qu'**elle se présente sous la forme d'une composition de soin et/ou de maquillage de matière(s) kératinique(s).

**52.** Utilisation cosmétique de particules selon l'une quelconque des revendications 1 à 39 à titre de véhicule d'actif(s) lipophile(s) dans une compositions cosmétique destinée au soin et/ou maquillage de matière(s) kératinique(s).

**53.** Utilisation de particules selon l'une quelconque des revendications 1 à 39 à titre de véhicule d'actif(s) lipophile(s) pour la préparation de compositions thérapeutiques.

**54.** Méthode de traitement cosmétique de matière(s) kératinique(s) comprenant au moins l'application sur une matière kératinique de particules selon l'une quelconque des revendications 1 à 39 ou d'une composition selon l'une quelconque des revendications 49 à 51.

FIGURE 1

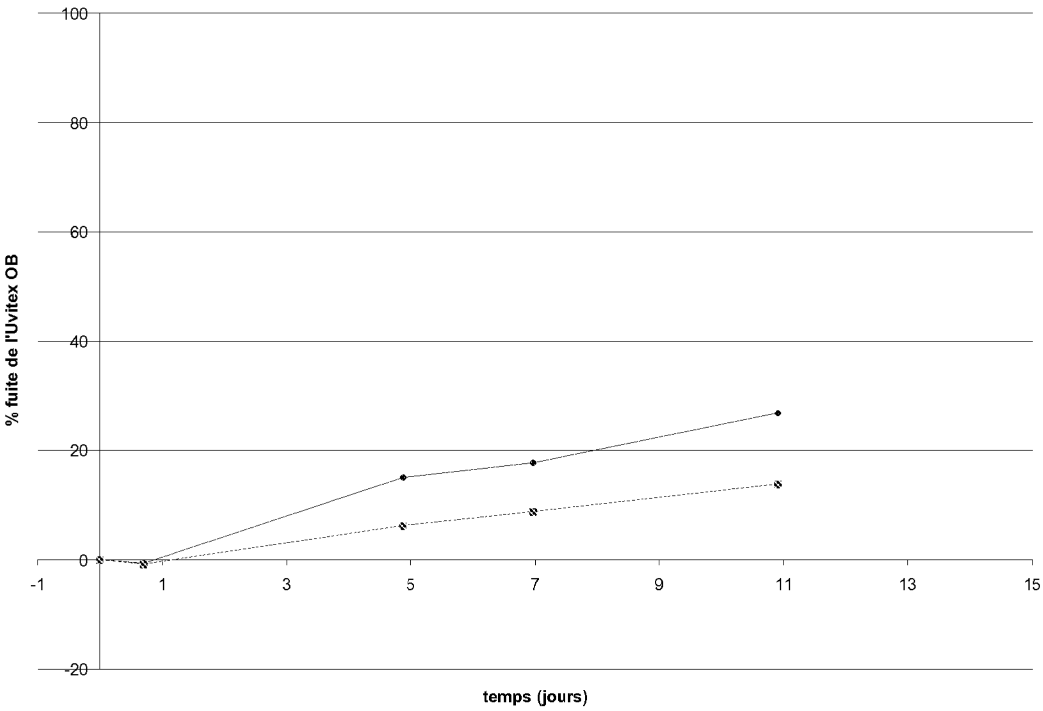


♦ (courbe supérieure) exemple 3▪

▪ (courbe inférieure) exemple 2 dilué 3 fois

Office européen des brevets

# RAPPORT DE RECHERCHE EUROPEENNE

Numéro de la demande
EP 07 12 3545

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | GB 2 416 524 A (DOW CORNING [US]) 1 février 2006 (2006-02-01)<br>* revendications 1,2,8 *<br>* page 2, ligne 17 *<br>* page 2, ligne 29 - page 3, ligne 9 *<br>* page 3, ligne 17 - ligne 18 *<br>* page 4, ligne 30 - ligne 32 *<br>* page 5, ligne 10 - ligne 14 *<br>* page 5, ligne 6 - ligne 7 *<br>* page 7, ligne 14 - ligne 17 *<br>* page 8, ligne 9 - ligne 15 *<br>* page 9, ligne 6 - ligne 15 *<br>* page 11, ligne 2 - ligne 6 *<br>----- | 1-54 | INV.<br>B01J13/14 |
| X | WO 02/20148 A (HENKEL KGAA [DE]; DREJA MICHAEL [DE]; RYBINSKI WOLFGANG [DE]; HOF MATT) 14 mars 2002 (2002-03-14)<br>* revendications 1,3,7,8,12,16,22,26,41,48,51 *<br>* page 7, ligne 8 - ligne 11 *<br>* page 8, ligne 25 - ligne 30 *<br>----- | 1-54 | |
| | | | DOMAINES TECHNIQUES RECHERCHES (IPC) |
| A | EP 0 436 450 A1 (RHONE POULENC CHIMIE [FR]) 10 juillet 1991 (1991-07-10)<br>* revendications 1,6 *<br>----- | 1-48 | B01J |

Le présent rapport a été établi pour toutes les revendications

| Lieu de la recherche | Date d'achèvement de la recherche | Examinateur |
|---|---|---|
| La Haye | 9 mai 2008 | Tarallo, Anthony |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..........
& : membre de la même famille, document correspondant

13
EPO FORM 1503 03.82 (P04C02)

## ANNEXE AU RAPPORT DE RECHERCHE EUROPEENNE RELATIF A LA DEMANDE DE BREVET EUROPEEN NO.

EP 07 12 3545

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

09-05-2008

| Document brevet cité au rapport de recherche | | Date de publication | Membre(s) de la famille de brevet(s) | | Date de publication |
|---|---|---|---|---|---|
| GB 2416524 | A | 01-02-2006 | AUCUN | | |
| WO 0220148 | A | 14-03-2002 | AU | 1815902 A | 22-03-2002 |
| | | | DE | 10044635 A1 | 04-04-2002 |
| EP 0436450 | A1 | 10-07-1991 | AU | 6849490 A | 04-07-1991 |
| | | | CA | 2033145 A1 | 28-06-1991 |
| | | | DE | 69007456 D1 | 21-04-1994 |
| | | | DE | 69007456 T2 | 20-10-1994 |
| | | | DK | 436450 T3 | 18-07-1994 |
| | | | ES | 2052224 T3 | 01-07-1994 |
| | | | FI | 906383 A | 28-06-1991 |
| | | | FR | 2656318 A1 | 28-06-1991 |
| | | | IE | 904372 A1 | 03-07-1991 |
| | | | JP | 4279664 A | 05-10-1992 |
| | | | JP | 7037572 B | 26-04-1995 |
| | | | NO | 905503 A | 28-06-1991 |
| | | | PT | 96327 A | 30-09-1991 |
| | | | US | 5232782 A | 03-08-1993 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 557489 A
- EP 447318 A
- WO 9325195 A
- WO 9305753 A
- FR 2864900
- EP 0274961 A
- WO 0196450 A
- GB 2407496 A
- EP 0465744 A
- EP 959066 A
- EP 1057849 A
- WO 9317060 A
- WO 9612754 A
- US 3175993 A
- US 4772675 A
- US 4871827 A
- US 4888380 A
- US 4898910 A
- US 4906719 A
- US 4962174 A
- EP 274961 A
- US 5364633 A
- US 5411744 A
- FR 2742677 A


### Littérature non-brevet citée dans la description


- **DONALD A. TOMALIA et al.** *Angewandte Chemie, Int. Engl. Ed.,* vol. 29 (2), 138-175
- **KUSABE.M.** *Pitture e Verniei - European Coating,* 2005, vol. 12-B, 43-49
- **PROBSTER, M.** *Adhesion-Kleben & Dichten,* 2004, vol. 481 (1-2), 12-14
- **LANDON, S.** *Pitture e Verniei,* 1997, vol. 73 (11), 18-24
- **HUANG, MOWO.** *Pitture e Verniei,* 2000, vol. 5, 61-67
- **EKWALL.** *Adv. Liq. Cryst.,* 1968, vol. 14
- **ROSERVEAR.** *JSCC,* 1968, vol. 19, 581
- **LACHAMPT ; VILA.** *Revue Française des Corps Gras,* 1969, (2), 87-111